# EUROPEAN PATENT APPLICATION

(11) **EP 2 377 532 A1**
(43) Date of publication of application: **19.10.2011**
(21) Application number: 11168684.6
(22) Date of filing: 18.08.2005
(51) Int. Cl.: A61K 31/401, A61K 31/404, A61K 31/41, A61K 31/4164, A61K 31/4166, A61K 31/4174, A61K 31/4178, A61K 31/4184, A61K 31/4245, A61K 31/47, A61K 31/472, A61K 31/502, A61K 31/554, A61K 38/06, A61K 38/05, A61P 17/00, A61P 17/06, A61P 17/02, A61P 35/00, A61K 38/07, A61K 38/08

(54) **Cosmetic and pharmaceutical compositions comprising ACE inhibitors and/or angiotensin II receptor antagonists for treating dermatological disorders**

(30) Priority: 18.08.2004 DK 200401247; 08.09.2004 US 607919 P
(62) Divisional of application: 05771000.6
(71) Applicant: Ace ApS, 3000 Helsingor (DK)
(72) Inventor: Bonnichsen, Richard, Victoria, Mahe (SC)
(74) Representative: Høiberg A/S

(57) **Abstract**

In one aspect, the present invention relates to use of an ACE inhibitor and/or angiotensin II receptor antagonist for the preparation of a medicament for the treatment of a dermatological disorder, particularly by topical application of said ACE inhibitor and/or angiotensin II receptor antagonist. The present invention also provides cosmetic methods for improving and/or maintaining the skin tone of an individual suffering from, or at risk of suffering from, a dermatological disorder, said method comprising contacting the skin of said individual with an ACE inhibitor and/or angiotensin II receptor antagonist.

## Description

This application claims priority from Danish patent application number PA 2004 01247 filed 18 August 2004, which is hereby incorporated by reference in its entirety. This application is a nonprovisional of U.S. provisional application Serial No. 60/607,919 filed 8 September 2004, which is hereby incorporated by reference in its entirety. All patent and non-patent references cited in the present application, are also hereby incorporated by reference in their entirety.

### Field of invention

The present invention provides compositions and methods for use in treatment and/or prevention of a dermatological disorder.

### Background of invention

### Skin

The skin dermis makes up 90% of the thickness of the skin and consists of a three-dimensional extracellular matrix (ECM) of loose connective tissue composed of highly stable fibers of collagen and elastin. Collagen is the major constituent of skin and constitutes more than 70% of the mass of the skin in terms of its dry weight. Collagens are synthesized by fibroblasts, and comprise a large family of glycoproteins which are located in the extracellular matrix. 20 different types of collagens (types I-XX) have been defined so far, and fibrillar collagens type I and II predominate in the skin (Epstein and Munderloh, J. Biol. Chem. 253:1336, 1978; Fukar et al., Acta Derm. Venereol. 68:196, 1988; Clore et al., Biochim. Biophys. Acta 586:384, 1979; Chan and Cole, Anal. Biochem. 139:322, 1984). In young and healthy skin, collagen molecules stay soluble and slide over one another, giving skin its softness, strength, resiliency and preventing skin tearing - a problem in diseased skin.

### Psoriasis

Psoriasis is a skin disease characterized by scaling and inflammation. Scaling occurs when cells in the outer layer of the skin reproduce faster than normal and pile up on the skin's surface. Psoriasis is often a chronic skin disease. There are several forms of psoriasis. The most common form is plaque psoriasis (its scientific name is psoriasis vulgaris). In plaque psoriasis, lesions have a reddened base covered by silvery scales. Other forms of psoriasis include:
Guttate Psoriasis: Drop-like lesions appear on the trunk, limbs, and scalp. Guttate psoriasis may be triggered by viral respiratory infections or certain bacterial (streptococcal) infections.
Pustular Psoriasis: Blisters of noninfectious pus appear on the skin. Attacks of pustular psoriasis may be triggered by medications, sunlight, infections, pregnancy, perspiration, emotional stress, or exposure to certain chemicals.
Inverse Psoriasis: Large, dry, smooth, vividly red plaques occur in the folds of skin near the genitals, under the breasts, or in the armpits. Inverse psoriasis is related to increased sensitivity to friction and sweating.
Erythrodermic Psoriasis: Widespread reddening and scaling of the skin is often accompanied by itching or pain. Erythrodermic psoriasis may be precipitated by severe sunburn, use of oral steroids (such as cortisone), or a drug-related rash.

### Ichthyosis:

Ichthyosis refers to a group of diseases characterized by excessive thickening of the stratum corneum, producing fish-like scales. The disease usually begins in early childhood, when the patient normally presents with white scales covering the trunk, extensor surfaces and face. Ichthyosis vulgaris is the most common type, however other forms also exist, such as, but not restricted to, Ichthyosis lamellaris, X-linked ichthyosis, Epidermolytic hyperkeratosis and/or Ichthyosis acquisita

### The renin-angiotensin-aldosterone system

The renin-angiotensin-aldosterone system plays an integral role in the pathophysiology of hypertension by affecting the regulation of fluid volume, electrolyte balance and blood volume. Renin catalyzes the conversion of angiotensinogen into an inactive substance, angiotensin I. Angiotensin-converting enzyme (ACE) then converts angiotensin I to the physiologically active angiotensin II, which binds the AT1 and/or AT2 angiotensin receptor and causes potent vasoconstriction, aldosterone secretion and sympathetic activation (Berstein KE, Berk BC, "The biology of angiotensin II receptors" Am J Kid Dis 1993). Current known angiotensin II receptor antagonists include losartan (Cozaar), valsartan (Diovan), irbesartan (Avapro), candesartan (Atacand), telmisartan (Micardis), eprosartan, tasosartan, zolarsartan, Isradipin, Candesartancilexetil and olmesartan medoxomil.

ACE inhibitors include Alacepril, Delapril, Cilazapril, Benazepril, Captopril, Enlapril, Fosinopril, Lisinopril, Moexipril, Perindopril, Ramipril, Pentopril, Zofenopril, Quinapril, Trandolapril, Imidapril, Isradipin, perindopril, spirapril, temocapril, Pentopril and Zofenopril and Zofenapril.

ACE inhibitors are used for treatment of hypertension, heart failure and nephropathy. Evidence suggests that angiotensin receptor antagonists may be just as effective as an angiotensin converting enzyme (ACE) inhibitor in treating patients who are at high risk of cardiovascular events after myocardial infarction (BMJ. 2003 Nov 15;327(7424):1123). ACE inhibitors may also slow the progress of diabetic kidney disease in middle-aged persons with type 2 diabetes (Annals of Internal Medicine 1999;131:660-667, 707-708.)

### Effect of the renin-angiotensin system on collagen

Angiotensin II stimulates collagen type I formation by activation of the collagen I gene (Tharaux PL, et al., "Angiotensin II activates collagen I gene through a mechanism involving the MAP/ER kinase pathway", Hypertension 2000, 36(3):330-336). Collagen type I gene expression increases after vascular injury, however using ACE inhibitors or angiotensin II antagonists decreases levels of gene expression significantly. (Patten RD et al., "Effects of angiotensin II receptor blockade versus angiotensin-converting-enzyme inhibition on ventricular remodelling following myocardial infarction in the mouse", Clin Sci (Lond). 2003 Feb;104(2):109-18). Activation of the renin-angiotensin-aldosterone system in the myocardial collagen network can lead to progressive collagen accumulation (Brilla CG et al., "Renin-angiotensin system and myocardial collagen matrix remodelling in hypertensive heart disease: in vivo and in vitro studies on collagen matrix regulation", Clin Investig 1993; 71 (5 Suppl):S35-41)

### Summary of invention

The skin provides protective functions of importance to our survival. These functions can be detrimentally affected by the changes in the skin structure due to dermatological pathological conditions or dermatological disorders. It is thus desirable to provide medicaments acting either prophylactically (i.e. to maintain skin structure and/or decrease the risk of a dermatological disorder) and/or by aiding restoration of damaged or abnormal skin structure. Furthermore, it is desirable for cosmetic reasons to improve the skin tone of an individual in cases when a dermatological condition causes, or could potentially cause, altered skin appearance. This altered skin appearance may not be damaging to physical health as such, but may affect psychological health, self-confidence, self-esteem (etc.) of the individual thus affected.

In particular, the skin's functions can be detrimentally affected by the changes in the skin structure due to fibroproliferative processes associated with diseased skin. It is thus desirable to provide medicaments for treatment of excessive fibroproliferative processes in the skin. For example, after tissue damage by various sources skin reacts by excessive reactive fibroproliferative reactions. Tissue may be damaged by free radicals i.e. from reperfusion injuries and inflammation. Various disorders of the skin also react by excessive fibroproliferative reactions. These damages and disease conditions can cause subcutaneous scar formation alternating with focus of tissue and collagen loss (wounds).

Furthermore, examination by scanning electron microscopy reveals a decrease in the number of collagen fibres in normal human skin with inflammatory disease states. It is thus desirable to provide medicaments for treatment of inflammatory disease states of the skin, which may lead to tissue damage which again can cause subcutaneous scar formation alternating with focus of tissue and collagen loss (wounds).

It has been found by the inventors of the present invention that skin diseases, such as skin diseases associated with inflammation or fibroproliferative processes, may be ameliorated or cured using one or more ACE inhibitor(s) and/or angiotensin II receptor antagonist(s). Thus, the present invention relates to use of one or more ACE inhibitor and/or angiotensin II receptor antagonist for the preparation of a medicament for the treatment or prophylaxis of a dermatological_disorder. In particular, it is preferred that said treatment or prophylaxis of a dermatological disorder is achieved by topical application of said one or more ACE inhibitor and/or angiotensin II receptor antagonist. Furthermore, in other aspects the present invention provides cosmetic methods for improving and/or maintaining the skin tone of an individual, and to the use of an ACE inhibitor and/or angiotensin II receptor antagonist for the preparation of a cosmetic composition

Without being bound by theory, it is hypothesised that the use of an ACE inhibitor or angiotensin inhibitor acts to even out the skin texture and aid restoration of skin structure. Surprisingly, the compounds used in the present invention do not lead to damaging levels of skin collagen reduction, but instead improve and/or maintain the skin tone of an individual.

Furthemore, without being bound by theory, it is believed that the healing processes caused by ACE-inhibition cause an organised regulation of repair in the various components of the matrix, thus reducing fibroproliferative processes and preventing formation of scars or other disordered tissue repair. This organised control of healing is also believed to be of value in skin diseases associated with inflammatory process, which may well lead to irregular scarring.

### Detailed description of the invention

ACE inhibitors and angiotensin II receptor antagonists suitable for use in any of the methods, uses and compositions of the present invention

Any suitable ACE inhibitor and/or angiotensin II receptor antagonist - or respective pharmaceutically/cosmetically acceptable salt thereof - may be used in any of the methods, uses and compositions of the present invention.

In one embodiment, said ACE inhibitor and/or angiotensin II receptor antagonist may be selected from the following, or a pharmaceutically acceptable salt thereof: Alacepril, Delapril, Benazepril, Cilazapril ,Captopril, Enlapril, Fosinopril, Lisinopril, Moexipril, Perindopril, Ramipril, Quinapril, Trandolapril, Imidapril, Isradipin, perindopril, spirapril, temocapril, Enalapril, losartan (Cozaar), valsartan (Diovan), irbesartan (Avapro), candesartan (Atacand), telmisartan (Micardis), eprosartan, tasosartan, zolarsartan, Zofenapril, Isradipin and Candesartancilexetil, or a cosmeceutically-acceptable salt thereof, alatriopril, altiopril calcium, ancovenin, benazepril, hydrochloride,benazeprilat, benzazepril, benzoylcaptopril, captoprilcysteine, captopriglutathione, ceranapril, ceranopril, ceronapril, cilazaprilat, converstatin, delapril-diacid, enalaprilat, enalkiren, enapril,epicaptopril, foroxymithine, fosfenopril, fosenopril, fosenopril sodium, fosinopril sodium, fosinoprilat, fosinoprilic acid, glycopril, hemorphin-4,idapril, indolapril, indolaprilat, libenzapril, lyciumin A, lyciumin B, mixanpril, moexiprilat, moveltipril, muracein A, muracein B, muracein C, perindoprilat, pivalopril, pivopril, quinapril hydrochloride, Pentopril, pentoprilat, quinaprilat, ramiprilat, spirapril, spirapril hydrochloride, spiraprilat, spiropril hydrochloride, temocapril hydrochloride, teprotide, trandolaprilat, utibapril, zabicipril, zabiciprilat, losartan (Cozaar), valsartan (Diovan), irbesartan (Avapro), candesartan (Atacand), telmisartan (Micardis), eprosartan, tasosartan, zolarsartan, Isradipin, Candesartancilexetil, olmesartan, medoxomil, zofenoprilat,
Asp-Arg-Val-Tyr-Val-His-Pro-Phe;
Asn-Arg-Val-Tyr-Val-His-Pro-Phe; Ala-Pro-Gly-Asp-Arg-Ile-Tyr-Val-His-Pro-Phe
Glu-Arg-Val-Tyr-Ile-His-Pro-Phe; Asp-Lys-Val-Tyr-Ile-His-Pro-Phe;
Asp-Arg-Ala-Tyr-Ile-His-Pro-Phe; Asp-Arg-Val-Thr-Ile-His-Pro-Phe;
Asp-Arg-Val-Tyr-Leu-His-Pro-Phe; Asp-Arg-Val-Tyr-Ile-Arg-Pro-Phe;
Asp-Arg-Val-Tyr-Ile-His-Ala-Phe; Asp-Arg-Val-Tyr-Ile-His-Pro-Tyr;
Pro-Arg-Val-Tyr-Ile-His-Pro-Phe; Asp-Arg-Pro-Tyr-Ile-His-Pro-Phe;
Asp-Ar-Val-Tyr; 2-Ile-His-Pro-Phe; Asp-Arg-norLeu-Tyr-Ile-His-Pro-Phe;
Asp-Arg-Val-Tyr-norLeu-His-Pro-Phe; Asp-Arg-Val-homoSer-Tyr-Ile-His-Pro-Phe;
Val-Trp;
or a pharmaceutically acceptable salt of any of the above-mentioned compounds.

Thus, said ACE inhibitor and/or angiotensin II receptor antagonist may be selected from Alacepril, Delapril , Cilazapril ,Benazepril, Captopril, Enlapril, Fosinopril, Lisinopril, Moexipril, Perindopril, Ramipril, Quinapril, Trandolapril, Imidapril, Isradipin, perindopril, spirapril, temocapril, Pentopril, pentoprilat, Enalapril, Zofenapril, or a pharmaceutically/cosmeceutically acceptable salt thereof. Equally preferably, said ACE inhibitor or angiotensin II receptor antagonist is selected from losartan (Cozaar), Val-Trp, valsartan (Diovan), irbesartan (Avapro), candesartan (Atacand), (Micardis), eprosartan, tasosartan, zolarsartan, Isradipin, Candesartancilexetil losartan (Cozaar), valsartan (Diovan), irbesartan (Avapro), candesartan (Atacand), telmisartan (Micardis), eprosartan, tasosartan, zolarsartan, Val-Trp, Isradipin, Candesartancilexetil and olmesartan medoxomil. Equally, further ACE inhibitors suitable for use in the present invention are any of those disclosed in Patent Application with publication number WO 00/56345 (incorporated herein by reference), such as any of the following:
Asp-Arg-Val-Tyr-Val-His-Pro-Phe
Asn-Arg-Val-Tyr-Val-His-Pro-Phe
Ala-Pro-Gly-Asp-Arg-Ile-Tyr-Val-His-Pro-Phe
Glu-Arg-Val-Tyr-Ile-His-Pro-Phe
Asp-Lys-Val-Tyr-Ile-His-Pro-Phe
Asp-Arg-Ala-Tyr-Ile-His-Pro-Phe
Asp-Arg-Val-Thr-Ile-His-Pro-Phe
Asp-Arg-Val-Tyr-Leu-His-Pro-Phe
Asp-Arg-Val-Tyr-Ile-Arg-Pro-Phe
Asp-Arg-Val-Tyr-Ile-His-Ala-Phe
Asp-Arg-Val-Tyr-Ile-His-Pro-Tyr
Pro-Arg-Val-Tyr-Ile-His-Pro-Phe
Asp-Arg-Pro-Tyr-Ile-His-Pro-Phe
Asp-Ar-Val-Tyr
2-Ile-His-Pro-Phe
Asp-Arg-norLeu-Tyr-Ile-His-Pro-Phe
Asp-Arg-Val-Tyr-norLeu-His-Pro-Phe
Asp-Arg-Val-homoSer-Tyr-Ile-His-Pro-Phe;
or a pharmaceutically acceptable salt of any of the above-mentioned compounds.

Other suitable ACE inhibitors for use in the present invention are disclosed in Oshima et al, "Peptide inhibitors of angiotensin I-converting enzyme in digests of gelatin by bacterial collagenase" (Biochem Biophys Acta 1979; 566: 128-137), and in Nakamura et al., "Purification and characterization of angiotensin I-converting enzyme inhibitors from a sour milk" (J Dairy Sci 1995; 78: 777-783) and Maruyama,S et al., "A peptide inhibitor of angiotensin I converting enzyme in the tryptic hydrolysate of casein", Agric.Biol.Chem. 46 (5), 1393-1394 (1982).

In one preferred embodiment of the present invention, the ACE inhibitor for use in any of the methods, uses and compositions of the present invention is a non-thiol-containing ACE-inhibitor (i.e. it does not contain a thiol group), or a pharmaceutically acceptable salt thereof.

In another preferred embodiment, the ACE inhibitor for use in any of the methods, uses and compositions of the present invention is a thiol-containing ACE-inhibitor (i.e. it comprises at least one thiol group), or a pharmaceutically acceptable salt thereof.

In one preferred embodiment of the present invention, the ACE inhibitor is lipophilic, such as selected from quinapril, quinaprilat, trandolaprilat, trandolapril, moexipril, moexiprilat, fosinoprilat, fosinopril, benazeprilat, benazepril, enalaprilat or enalapril, or a pharmaceutically acceptable salt thereof. More preferably, said ACE inhibitor is selected from quinaprilat, trandolaprilat, moexiprilat, fosinoprilat, benazeprilat or enalaprilat. In another preferred embodiment of the present invention, the ACE inhibitor is non-lipophilic, such as selected from the following: captopril, lisinopril, ramaprilat or ramapril, or a pharmaceutically acceptable salt thereof.

In one preferred embodiment of the present invention, the ACE inihibitor binds to the zinc-binding ligand of the active site of ACE via a sulfhydryl group, such as captopril, zofenopril and/or alacepril, or a pharmaceutically acceptable salt thereof.

In another preferred embodiment of the present invention, said ACE inhibitor binds to the zinc-binding ligand of the active site of ACE via a phosphinyl group, such as fosinopril or a pharmaceutically acceptable salt thereof. In another preferred embodiment of the present invention, said ACE inhibitor binds to the zinc-binding ligand of the active site of ACE via a carboxyl group, such as ramipril or lisinopril, or a pharmaceutically acceptable salt thereof.

In a preferred embodiment, the ACE inhibitors for use in the present invention are selected from captopril, enalaprilat, lisinopril, benazeprilat, fosinoprilat, moexiprilat, ramiprilat, trandolaprilat or quinaprilat, or a pharmaceutically acceptable salt thereof. More preferably, said ACE inhibitor is ramiprilat or lisinopril. Alternatively, said ACE inhibitor is lisinopril or captopril. Most preferably, the ACE inhibitor is lisinopril.

Most ACE-inhibitors are prodrugs that require activation through hepatic biotransformation. Thus, for topical application, it is preferred that the biologically active form of the ACE inhibitor is administered (e.g. as a salt), such as e.g. any of the the following compounds: quinaprilat, trandolaprilat, moexiprilat, fosinoprilat, benazeprilat, enalaprilat or ramaprilat, or a pharmaceutically acceptable salt thereof.

It is to be understood herein that when an ACE inhibitor or angiotensin II receptor antagonist is mentioned herein for topical use, the biologically active form of this compound may also be substituted for precisely the same use.

Ramiprilat is one preferred drug for local application. It has well-known pharmacological properties and it has been used systemically in humans for decades. It has very modest and tolerable side effects.

ACE inhibitors suitable for use in the present invention are also any of those disclosed in Patent Application with publication number WO 00/56345, incorporated herein by reference. Other suitable ACE inhibitors for use in the present invention are disclosed in Oshima et al, "Peptide inhibitors of angiotensin I-converting enzyme in digests of gelatin by bacterial collagenase" (Biochem Biophys Acta 1979; 566: 128-137), and in Nakamura et al., "Purification and characterization of angiotensin I-converting enzyme inhibitors from a sour milk" (J Dairy Sci 1995; 78: 777-783) and Maruyama,S et al., "A peptide inhibitor of angiotensin I converting enzyme in the tryptic hydrolysate of casein", Agric.Biol.Chem. 46 (5), 1393-1394 (1982).

### Cosmetic composition

In one aspect of the present invention disclosed herein, a cosmetic composition is provided. Said cosmetic composition is suitable for use in any of the cosmetic methods or uses described herein. The cosmetic composition comprises at least one ACE inhibitor and/or angiotensin II receptor antagonist, or a cosmeceutically acceptable salt thereof. By "ACE inhibitor" is meant any substance capable of inhibiting, fully or partially, the biological functions of ACE. By angiotensin II receptor antagonist is meant any substance capable of antagonising, fully or partially, the biological functions of an angiotensin receptor. Preferably, said ACE inhibitor and/or angiotensin II receptor antagonist is selected from any of the groups described in the section above, entitled: "ACE inhibitors and angiotensin II receptor antagonists suitable for use in any of the methods, uses and compositions of the present invention", or a cosmeceutically-acceptable salt thereof. In one embodiment of the present invention, said composition comprises more than one ACE inhibitor or angiotensin II receptor antagonist, or a cosmeceutical salt thereof.

Preferably, said ACE inhibitor or angiotensin II receptor antagonist is present in an concentration between about 0.01 mg/kg-100 mg/kg, such as 0.1 mg/kg-90 mg/kg, such as 0.5 mg/kg-75 mg/kg, such as 1 mg/kg-60 mg/kg, such as 2 mg/kg-45mg/kg, such as 5 mg/kg-30 mg/kg, such as 10 mg/kg-15 mg/kg, such as 10 mg/kg-12 mg/kg. In another preferred embodiment, said ACE inhibitor or angiotensin II receptor antagonist is present in an amount between about 0.1 mg/kg-10 mg/kg.

In one preferred embodiment of the present invention, the cosmetic compositions according to the invention can take the form of any suitable cosmetic product. Preferably, the compositions take the form of a care, treatment, cleaning or protection product for the face or the body skin, including the scalp, such as a day and/or night and/or hydrating care composition for the face or the body; an anti-wrinkle or anti-ageing composition for the face; a composition for irritated skins; a make-up removing composition; a body milk, a sun protective, artificial sun tanning (self-tanning) or after-sun care composition; a sun protective cream or gel; a face skin, body or lip makeup product, such as a foundation cream, a tinted cream, a cheek or eye-lid makeup product, a free or compact powder, an anti eye-ring stick, a concealing stick, a lipstick or a lip care product.

The composition may also be applied to any part of the human body where skin maintenance or improvement benefits are desired, such as for example one or more of: hand, foot, limb, arm, leg, torso, back, chest, face, scalp, head area etc.

### Cosmetic method

Many dermatological disorders result in altered skin appearance during the course of pathology, and pathologically-induced changes in skin appearance may still be present after the disease process itself is over. This altered skin appearance to be treated in the case of the cosmetic method disclosed herein may not be damaging to physical health as such, but may affect psychological health, self-confidence, self-esteem etc. Thus, one aspect of the present invention relates to a cosmetic method for improving and/or maintaining the skin tone of an individual suffering from, having suffered from, or at risk of suffering from, a dermatological disorder, said method comprising contacting the skin of said individual with an ACE inhibitor and/or angiotensin II receptor antagonist.

By "skin tone" herein is meant any aspect of the skin, such as the texture, suppleness, moisture levels, radiance, smoothness and surface appearance, although preferably not the presence or absence of wrinkles and/or fine lines, or signs of natural or accelerated ageing processes. By "improving and/or maintaining the skin tone" is meant any process in which any undesirable changes in the skin tone are lessened or even prevented. It should however be underlined that such aspects not relating to the skin layers themselves, i.e:
(i) hair growth (or lack of it)
(ii) discharge of sebum from the sebaceous glands and/or acne
(iii) growth of the nails
(iv) lymphatic drainage
(v) fatty mass
(vi) water retention and/or local oedemas
(vii) sodium imbalance and/or local oedemas
are preferably excluded from the definition of "skin tone" as used herein.

It is preferred that the cosmetic method provided herein is for reducing uneven collagen deposits present in diseased skin, thus evening out the skin texture. In one preferred embodiment, by "improving and/or maintaining the skin tone" is meant countering undesirable skin changes not caused by ageing processes. For example, the uses and methods of the present invention can cause: reducing or preventing undesirable skin fibrosis caused by a pathological condition and/or reducing undesirable scarring after skin inflammation due to a pathological condition and/or increasing suppleness of inflamed skin, which is preferably inflamed due to a pathological process.

The compositions of the invention can be applied to the skin on an as-needed basis, for example, they can be applied to the skin in the morning and/or in the evening, for instance every evening, and/or during the day. It is preferred that topical application be once a month to about 7 or 8 times daily, preferably from about 7 times a week to about 4 times a day, most preferably about once or twice a day. In another preferred embodiment, for an intensive treatment programme the compositions of the invention can be applied on a frequent basis throughout the day and/or night, such as 8 times daily.

To maintain the beneficial effects of the composition on the skin, it is preferred that the cosmetic method comprises repeatedly performing said contacting over an extended period of time, preferably over the lifetime of the user, equally preferably over a period from 4 weeks to twenty years, more preferably from about 6 months to about five years, resulting in the improvement and/or maintenance of an individual's skin tone. In another preferred embodiment of the present invention, the contacting is conducted at least once daily.

In one preferred embodiment of the present invention, the individual is a postmenopausal, female human being. In another, equally preferred embodiment, said individual is a pre-menopausal, female human being. In another, equally preferred embodiment, said individual is a male human being.

In one preferred embodiment, the cosmetic methods disclosed herein may further comprise contacting the skin with one or more other compound, as described in the "combinations" section below.

### Use of an ACE inhibitor or angiotensin II receptor antagoniser for the preparation of a medicament

By "treatment" when discussing medical methods and practices herein, is meant one or more of prophylaxis, cure, lessening of pathological symptoms or other beneficial effect on an individual suffering from, or at risk of suffering from, a pathological condition.

In one aspect of the invention, the present invention relates to use of an ACE inhibitor and/or angiotensin II receptor antagonist for the preparation of a medicament for the treatment of a dermatological disorder. By "ACE inhibitor" is meant any substance capable of inhibiting, fully or partially, the biological functions of ACE. By angiotensin II receptor antagonist is meant any substance capable of antagonising, fully or partially, the biological functions of an angiotensin receptor. Preferably, said ACE inhibitor and/or angiotensin II receptor antagonist is selected from any of the groups described in the section above, entitled: "ACE inhibitors and angiotensin II receptor antagonists suitable for use in any of the methods, uses and compositions of the present invention", or a pharmaceutically-acceptable salt thereof. In one embodiment of the present invention, said composition comprises more than one ACE inhibitor or angiotensin II receptor antagonist, or a cosmeceutical salt thereof.

By "dermatological disorder" is meant a pathological condition affecting the skin, for example any instance in which the skin structure of the individual is detrimentally altered, such as by inflammation processes or alterations to the skin's structural layers. Said dermatological disorder may have been directly caused by a pathological process originating in the skin itself, or may be a symptom of a pathological disorder originating elsewhere within the individual's body.

Preferably, said dermatological disorder is associated with skin inflammation and/or is associated with excessive fibroproliferative activation.

Thus, in one embodiment of the present invention, said dermatological disorder is associated with inflammation. For example, the present invention may be used for treating a disorder selected from dermatitis or eczema, such as any of the dermatitis or eczema embodiments described herein below. Preferably, said dermatological disorder associated with inflammation is a non-tumourous pathological condition of the skin, such as a disease of the epidermis, such as any of the embodiments described herein below. For example, said disorder can be a blistering skin disorder, such as selected from the group consisting of: Impetigo, Staphylococcal scalded skin syndrome, Subcorneal pustular dermatosis of Sneddon-Wilkinson, Pemphigus vulgaris, Pemphigus vegetans, Pemphigus erythematosus Senear-Usher, Pemphigus foliaceus, Paraneoplastic pemphigus, Hailey-Hailey (Familiar benign pemphigus), Transient acantholytic dermatosis (of Grover), Herpes zoster, varicella, Herpes simplex, Herpes zoster, varicella; Bullous pemphigoid, Cicatricial pemphigoid, Pemphigoid (herpes) gestationis, Porphyria cutanea tarda, Dermatitis herpetiformis Duhring, IgA linear dermatosis, Erythema multiforme, Toxic epidermal necrolysis of Lyell, Stevens-Johnson syndrome, Graft versus host reaction, Epidermolysis bullosa (such as Epidermolysis bullosa dystrophica and/or Epidermolysis bullosa acquisita), acute Allergic contact dermatitis, Dyshidrotic dermatitis, Eczema atopicum (dermatitis atopica), Subacute and chronic (eczem) dermatitis, Microbial eczema, Granuloma gluteale infantum, acute Toxic contact (irritative) dermatitis, chronic Toxic contact (irritative) dermatitis, Seborrhoic dermatitis, Pityriasis amiantacea.

Said disorder associated with inflammation can alternatively be a dermatological disorder caused by rubbing the skin and/or scratching the skin and/or pressure on the skin, such as any of the embodiments of this disorder described herein below.

In another preferred embodiment, said disorder associated with inflammation is a dermatological disorder caused by infiltration of the upper and/or deep corium, such as any of the embodiments of a dermatological disorder caused by infiltration of the upper and/or deep corium described herein below.

In another preferred embodiment, said disorder associated with inflammation is a dermatological disorder caused by one or more vasculitides, such as any of the disorders associated with vasculitides described herein below.

In another preferred embodiment, said disorder associated with inflammation is a dermatological disorder caused by one or more panniculitides, such as any of the disorders caused by one or more panniculitides described herein below.

In another preferred embodiment, said disorder associated with inflammation is a dermatological disorder caused by bacterial disease, such as any of the dermatological disorders caused by bacterial disease described herein below.

In another preferred embodiment, said disorder associated with inflammation is a dermatological disorder caused by myotic disease, such as any of the embodiments of a dermatological disorder caused by myotic disease described herein below.

In another preferred embodiment, said disorder associated with inflammation is a dermatological disorder caused by parasitic, insect-borne or viral disease, such as any of the embodiments of parasitic, insect-borne or viral diseases described herein below.

In another preferred embodiment, said disorder associated with inflammation is a dermatological disorder caused by a disease of the hair follicles, such as any of the embodiments of at dermatological disorder caused by a disease of the hair follicles described herein below.

In another preferred embodiment, said disorder associated with inflammation is a dermatological disorder caused by a disease of the nails, such as any of the nail diseases described herein below.

In another preferred embodiment, said disorder associated with inflammation is a dermatological disorder caused by a disease of the sweat glands, such as any of the sweat gland diseases described herein below.

In another preferred embodiment, said disorder associated with inflammation is a dermatological disorder caused by an exogenic alteration of the skin, such as any of the dermatological disorders caused by an exogenic alteration of the skin described herein below.

In another preferred embodiment, said disorder associated with inflammation is a dermatological disorder caused by a metabolic and/or nutrition deficiency or imbalance, such as any of the dermatological disorders caused by a metabolic and/or nutrition deficiency or imbalance described herein below.

In another preferred embodiment, said disorder associated with inflammation is a dermatological disorder caused by a drug reaction, such as any of the dermatological disorders caused by a drug reaction described herein below.

In another preferred embodiment, said disorder associated with inflammation is a dermatological disorder caused by Organoid nevi and/or malformations of the skin, such as any of the dermatological disorders caused by Organoid nevi and/or malformations of the skin described herein below.

In another preferred embodiment, said disorder associated with inflammation is a dermatovenereal disease, such as any of the dermatovenereal diseases described herein below.

In another aspect, the present invention is related to use of one or more ACE inhibitor and/or angiotensin II antagonist in the treatment of a dermataological disease associated with excessive fibroproliferative activation. Thus, the present invention may be used to treat a dermatological disorder caused by disorders of melanin pigmentation, such as any of the dermatological disorders caused by disorders of melanin pigmentation described herein below.

In another preferred embodiment of the treatment of a dermataological disease associated with excessive fibroproliferative activation, said dermatological disease is tumourous skin disorder. Said tumourous skin disorder can be a benign epidermal tumor, such as any of the benign epidermal tumors described herein below.

In another preferred embodiment of the present invention, said tumourous skin disorder is a dermatological disorder caused by Tumorous infiltration of the corium, such as any of the disorders caused by Tumorous infiltration of the corium described herein below.

In another preferred embodiment of the present invention, said tumourous skin disorder is a malignant epidermal tumor, such as any of the malignant epidermal tumors described herein below.

In another preferred embodiment of the present invention, said tumourous skin disorder is an adnexal tumor, preferably a tumour of the hair follicle, such as any of the adnexal tumors described herein below.

In another preferred embodiment of the present invention, said tumourous skin disorder is a tumour of the sweat glands, such as any of the sweat gland tumours described herein below.

In another preferred embodiment of the present invention, said tumourous skin disorder is a sebaceous tumor, such as any of the sebaceous tumors described herein below.

In another preferred embodiment of the present invention, said tumourous skin disorder is a mesenchymal tumor, such as any of the mesenchymal tumors described herein below.

In another preferred embodiment of the present invention, said tumourous skin disorder is a melanocytic tumor, such as any of the melanocytic tumors described herein below.

In another preferred embodiment of the present invention, said tumourous skin disorder is a Merkel cell carcinoma, such as any of the Merkel cell carcinomas described herein below.

In another preferred embodiment of the present invention, said tumourous skin disorder is a cyst of the skin and/or subcutis, such as any of the cysts of the skin and/or subcutis described herein below.

In another preferred embodiment of the present invention, the dermatological disease associated with excessive fibroproliferative activation is Ichthyosis, such as selected from Ichthyosis vulgaris, Ichthyosis lamellaris, X-linked ichthyosis, Epidermolytic hyperkeratosis and/or Ichthyosis acquisita.

In another preferred embodiment of the present invention, the dermatological disease associated with excessive fibroproliferative activation is Psoriasis, such as selected from the group consisting of: psoriatic erytroderma, Palmoplantar psoriasis, palmoplantar pustulosis, generalized pustular psoriasis of Zumbusch and/or Lingua geographica.

In another preferred embodiment of the present invention, the dermatological disease associated with excessive fibroproliferative activation is Porokeratosis, such as any of the Porokeratosis described herein below.

In another preferred embodiment of the present invention, the dermatological disease associated with excessive fibroproliferative activation is a dermatological disorder caused by perivascular infiltration of the upper dermis, such as any of the dermatological disorders caused by perivascular infiltration of the upper dermis described herein below.

In another preferred embodiment of the present invention, the dermatological disease associated with excessive fibroproliferative activation is a dermatological disorder caused by diffuse infiltration of the upper dermis, such as any of the dermatological disorders caused by diffuse infiltration of the upper dermis described herein below.

In another preferred embodiment of the present invention, the dermatological disease associated with excessive fibroproliferative activation is a dermatological disorder caused by granulomatous processes of the deeper corium, such as any of the dermatological disorders caused by granulomatous processes of the deeper corium described herein below.

In another preferred embodiment of the present invention, the dermatological disease associated with excessive fibroproliferative activation is a dermatological disorder caused by a connective tissue disorder, such as any of the dermatological disorders caused by a connective tissue disorder described herein below.

In another preferred embodiment of the present invention, the dermatological disease associated with excessive fibroproliferative activation is a disorder of the elastic fibres, such as any of the disorders of the elastic fibres described herein below.

In another preferred embodiment of the present invention, the dermatological disease associated with excessive fibroproliferative activation is a dermatological disorder caused by deposition of foreign material in the dermis, such as any of the dermatological disorders caused by deposition of foreign material in the dermis described herein below.

### Examples of specific dermatological diseases or disorders treatable using the compositions and methods of the present invention

In one preferred embodiment of the present invention, the dermatological disorder is caused by, or associated with, a non-tumourous pathological condition of the skin. In one preferred embodiment of the invention, said non-tumourous pathological condition of the skin is a disease of the epidermis. Preferably, said skin diseases is selected from one or more of the following:
- Ichthyosis (such as Ichthyosis vulgaris, Ichthyosis lamellaris, X-linked ichthyosis, Epidermolytic hyperkeratosis and/or Ichthyosis acquisita)
- Psoriasis (such as psoriatic erytroderma, Palmoplantar psoriasis, palmoplantar pustulosis, generalized pustular psoriasis of Zumbusch and/or Lingua geographica,)
- Darier's disease
- Porokeratosis
- Pityriasis rubra pilaris

Thus, in one preferred embodiment of the present invention, psoriasis or Ichthyosis is treated. Said psoriasis may be any form of psoriasis, such as e.g. plaque psoriasis. Other forms of psoriasis suitable for treatment using the methods and compositions of the present invention include, but are not restricted to: Guttate Psoriasis, Pustular Psoriasis, Inverse Psoriasis, and Erythrodermic Psoriasis. In another preferred embodiment of the present invention, the psoriasis treated is selected from the group consisting of: psoriatic erytroderma, Palmoplantar psoriasis, palmoplantar pustulosis, generalized pustular psoriasis of Zumbusch and/or Lingua geographica. Said Ichthyosis treated by compositions and methods of the present invention may be any form of Ichthyosis, such as Ichthyosis vulgaris, Ichthyosis lamellaris, X-linked ichthyosis, Epidermolytic hyperkeratosis and/or Ichthyosis acquisita.

In another preferred embodiment of the invention, the non-tumourous pathological condition of the skin treated using the methods and compositions described herein is a blistering skin disorder, preferably selected from one or more of the following:
- Impetigo
- Staphylococcal scalded skin syndrome
- Subcorneal pustular dermatosis of Sneddon-Wilkinson
- Pemphigus vulgaris
- Pemphigus vegetans
- Pemphigus erythematosus Senear-Usher
- Pemphigus foliaceus
- Paraneoplastic pemphigus
- Hailey-Hailey (Familiar benign pemphigus)
- Transient acantholytic dermatosis (of Grover)
- Herpes zoster, varicella, Herpes simplex
- Herpes simplex
- Herpes zoster, varicella
- Bullous pemphigoid
- Cicatricial pemphigoid
- Pemphigoid (herpes) gestationis
- Porphyria cutanea tarda
- Dermatitis herpetiformis Duhring
- IgA linear dermatosis
- Erythema multiforme
- Toxic epidermal necrolysis of Lyell
- Stevens-Johnson syndrome
- Graft versus host reaction
- Epidermolysis bullosa (such as Epidermolysis bullosa dystrophica and/or Epidermolysis bullosa acquisita)
- Allergic contact dermatitis, acute
- Dyshidrotic dermatitis
- Eczema atopicum (dermatitis atopica)
- Subacute and chronic (eczem) dermatitis
- Microbial eczema
- Granuloma gluteale infantum
- Toxic contact (irritative) dermatitis, acute
- Toxic contact (irritative) dermatitis, chronic
- Seborrhoic dermatitis
- Pityriasis amiantacea

In another preferred embodiment of the invention, said non-tumourous pathological condition of the skin is a dermatological disorder caused by rubbing the skin and/or scratching the skin and/or pressure on the skin, preferably selected from one or more of the following:
- Lichen simplex
- Prurigo nodularis
- Prurigo senilis
- Atopic dermatitis
- Excoriation
- Chronic pressure (e.g. on hip)
- Amputation stump
- Granuloma fissuratum
- Pressure necrosis
- Tyloma, callus
- Clavus

In another preferred embodiment of the invention, said non-tumourous pathological condition of the skin is a dermatological disorder caused by perivascular infiltration of the upper dermis, preferably selected from one or more of the following:
- Pityriasis lichenoides Mucha Habermann
- Pityriasis lichenoides acuta
- Pityriasis lichenoides chronica
- Pityriasis rosea Gibert

In another preferred embodiment of the invention, said non-tumourous pathological condition of the skin is a dermatological disorder caused by diffuse infiltration of the upper dermis, preferably selected from one or more of the following:
- Lichen ruber planus
- Lichen verrucosus (hypertrophicus)
- Lichen ruber, vesicular
- Lichen planopilaris
- Lichen planus like keratosis (benign lichenoid keratosis)
- Lichenoid drug reaction
- Lichen nitidus
- Lichen striatus
- Erythema dyschromicum perstans

In another preferred embodiment of the invention, said non-tumourous pathological condition of the skin is a dermatological disorder caused by infiltration of the upper and/or deep corium, preferably selected from one or more of the following:
- Erythema annulare centrifugum (erythema figuratum)
- Urticaria
- Pruritic urticarial papules and plaques of pregnancy (PUPPP)
- Granuloma faciale (eosinophilicum)
- Sweet's syndrome
- Pyoderma gangrenosum

In another preferred embodiment of the invention, said non-tumourous pathological condition of the skin is a dermatological disorder caused by granulomatous processes of the deeper corium, preferably selected from one or more of the following:
- Granuloma annulare
- Granuloma annulare, deep
- Annular elastolytic granuloma
- Necrobiosis lipoidica
- Rheumatoid nodule
- Chondrodermatitis nodularis chronica helicis
- Sarcoidosis
- Erythema induratum Bazin
- Foreign body granulomas
- Cat scratch disease

In another preferred embodiment of the invention, said non-tumourous pathological condition of the skin is a dermatological disorder caused by a connective tissue disorder, preferably selected from one or more of the following:
- Lupus erythematosus
- Lupus erythematodes, acute
- Lupus erythematodes, subacute
- Lupus erythematodes, chronic
- Lupus erythematodes profundus
- Dermatomyositis
- Osteoarthritis, Heberden nodes
- Scleroderma, morphea
- Diffuse scleroderma
- Localized scleroderma, morphea
- Lichen sclerosus et atrophicus
- Eosinofilic fasciitis
- Fibromatosis
- Striae distensae
- Skin graft

In another preferred embodiment of the invention, said non-tumourous pathological condition of the skin is a dermatological disorder caused by a disorder of the elastic fibres, preferably selected from one or more of the following:
- Anetoderma
- Actinic degeneration of the corium
- Pseudoxanthoma elasticum

In another preferred embodiment of the invention, said non-tumourous pathological condition of the skin is a dermatological disorder caused by one or more vasculitides, preferably selected from one or more of the following:
- Leukocytoclastic vasculitis
- Other vasculitides (such as Polyarteritis nodosa, Purpura, Urticarial vasculitis or Vasculitis and/or mycotic sepsis)
- Chronic venous insufficiency (such as Venous varices and/or Ulcus cruris
- Cutis marmorata
- Cryoglobulinemia (such as Cryoglobulinemia in monoclonal gamapathy and/or Mixed cryoglobulinemia)
- Livedo vasculitis
- Acrocyanosis
- Lymphedema
- Lymphangitis (such as Acute lymphangitis and/or Sclerosing lymphangitis of the penis)

In another preferred embodiment of the invention, said non-tumourous pathological condition of the skin is a dermatological disorder caused by one or more panniculitides, preferably selected from one or more of the following:
- Erythema nodosum
- Panniculitis arteficialis (factitial)
- Calcifying panniculitis
- Panniculitis caused by cold
- Eosinophilic panniculitis
- Lipomembranous panniculitis
- Traumatic panniculitis
- Panniculitis caused by steroids
- Pancreatic panniculitis
- Other panniculitides

In another preferred embodiment of the invention, said non-tumourous pathological condition of the skin is a dermatological disorder caused by deposition of foreign material in the dermis, preferably selected from one or more of the following:
- Amyloidosis (such as Systemic (secondary) amyloidosis)
- Mucinoses (such as one or more of: Pretibial myxedema, Reticular erythematous mucinosis, Papular mucinosis, Scleredema, Scleromyxedema and/or Myxoma)
- Calcium deposits (such as one or more of: Chronic dermatomyositis, Subepidermal calcifying nodule, Calcinosis scroti, Calciphylaxis and/or Dystrofic dermal calcifications
- Osteoma cutis (such as Progressive osseous heteroplasia)
- Gout, arthritis uratica
- Hemosiderin
- Tatoo (such as Amalgam tatoo)
- Argyrosis
- Deposits of corticosteroids in the dermis
- Ochronosis
- Colloid milium
- Subcutaneous emphysema

In another preferred embodiment of the invention, said non-tumourous pathological condition of the skin is a dermatological disorder caused by bacterial disease, preferably selected from one or more of the following:
- Bacterial inflammations with suppuration, pyodermia (such as Erysipelas, Ecthyma, Angulus infectiosus, Aphta, Phlegmone, gangrene, Erythrasma, Pitted keratolysis, Borreliosis, Erythema chronicum migrans)
- Mycobacteria, actinomycetes, such as Tuberculosis cutis, Scrofuloderma, Lupus vulgaris, Reaction to BCG vaccination or sensitivity tests, Papulonecrotic tuberculid, diseases caused by atypical mycobacteria, Leprosy and/or Actinomycosis
- Rhinoscleroma

In another preferred embodiment of the invention, said non-tumourous pathological condition of the skin is a dermatological disorder caused by myotic disease, preferably selected from one or more of the following:
- Superficial tinea
- Candidosis
- Tinea nigra
- Dermatophyton
- Follicular mycosis
- Deep mycotic processes (such as Cryptococcosis, Sporotrichosis)
- Onychomycosis
- Paronychia candidomycetica

In another preferred embodiment of the invention, said non-tumourous pathological condition of the skin is a dermatological disorder caused by parasitic, insect-borne or viral disease, preferably selected from one or more of the following:
- Leishmaniasis
- Demodecidosis
- Scabies
- Larva migrans
- Pediculosis capitis
- Pediculosis pubis
- Insect bite reaction
- Tick
- Hand, foot and mouth disease

In another preferred embodiment of the invention, said non-tumourous pathological condition of the skin is a dermatological disorder caused by a disease of the hair follicles, preferably selected from one or more of the following:
- Acne vulgaris
- Rosacea
- Dermatitis perioralis
- Alopecia
- Alopecia areata
- Diffuse alopecia
- Defluvium
- Pseudopelade of Brocq
- Alopecia in lupus erythematodes
- Follicular mucinosis (alopecia mucinosa) (such as Primary follicular mucinosis and/or Secondary follicular mucinosis in mycosis fungoides)
- Alopecia androgenica
- Trichotillomania
- Purulent folliculitis
- Folliculitis keloidalis nuchae
- Ostiofolliculitis (superficial folliculitis)
- Perforating folliculitis
- Eosinophilic pustular folliculitis
- Keratosis pilaris
- Lichen spinulosus
- Ulerythema ophryogenes

In another preferred embodiment of the invention, said non-tumourous pathological condition of the skin is a dermatological disorder caused by a disease of the nails, preferably selected from one or more of the following:
- Posttraumatic onycholysis
- Psoriatic onycholysis
- Onychoschisis
- Onychophagia
- Pterygium unguis
- Unguis incarnatus

In another preferred embodiment of the invention, said non-tumourous pathological condition of the skin is a dermatological disorder caused by a disease of the sweat glands, preferably selected from one or more of the following:
- Hidradenitis axillaris (suppurativa)
- Eccrine hidradenitis with neutrophils
- Necroses of eccrine sweat glands
- Myoclonic epilepsy
- Clear cell metaplasia of eccrine ducts
- Syringomucinous metaplasia
- Mucinous metaplasia

In another preferred embodiment of the invention, said non-tumourous pathological condition of the skin is a dermatological disorder caused by an exogenic alteration of the skin, preferably selected from one or more of the following:
- Cauterisatio
- Combustio (burns)
- Congelatio (frostbite)
- Livedo e calore, erythema ab igne
- Dermatitis artefacta
- Haematoma
- Radiodermatitis (such as acute or chronic radiodermatitis)
- Phototoxic and photoallergic reactions
- Syndrome Favre-Racouchot

In another preferred embodiment of the invention, said non-tumourous pathological condition of the skin is a dermatological disorder caused by a metabolic and/or nutrition deficiency or imbalance, such as pellagra

In another preferred embodiment of the invention, said non-tumourous pathological condition of the skin is a dermatological disorder caused by a drug reaction, preferably selected from one or more of the following:
- Lichenoid drug eruptions
- Fixed drug reaction
- Toxoallergic reaction
- Pustular drug reaction
- Necrotising drug reaction
- Striae after prolonged therapy by corticosteroids

In another preferred embodiment of the invention, said non-tumourous pathological condition of the skin is a dermatological disorder caused by Organoid nevi and/or malformations of the skin, preferably selected from one or more of the following:
- Nevus sebaceus (Jadassohn)
- Epidermal nevus
- Ichthyosis hystrix (widespread linear epidermal nevus)
- Inflammatory linear verrucous epidermal nevus (ILVEN)
- Nevus comedonicus
- Familial dyskeratotic comedones
- White sponge nevus of oral mucosa
- Nevus lipomatosus

In another preferred embodiment of the invention, said non-tumourous pathological condition of the skin is a dermatological disorder caused by disorders of melanin pigmentation, preferably selected from one or more of the following:
- Vitiligo
- Hypomelanosis guttata
- Pityriasis alba
- Melasma, chloasma
- Ephelides, freckles
- Melanocytic macule of the lip
- Dowling-Degos disease
- Nevus achromicus

In another preferred embodiment of the present invention, an ACE inhibitor and/or angiotensin II receptor antagonist is used for the preparation of a medicament for the treatment of a dermatological disorder caused by, or associated with, a tumourous skin disorder. In one preferred embodiment of the invention, said tumourous skin disorder is a benign epidermal tumor, preferably selected from one or more of the following:
- Seborrhoic keratosis
- Melanoacanthoma
- Dermatosis papulosa nigra
- Lentigo solaris
- Melanosis of Becker (Becker's nevus)
- Stucco keratosis
- Acrokeratosis verruciformis Hopf
- Confluent and reticulated papillomatosis
- Acanthosis nigricans
- Keratotic processes (such as Multiple minute digitate keratoses, Postirradiation hyperkeratoses, Minute aggregate keratoses and/or Waxy dermatosis)
- Warty dyskeratoma (solitary Darier's disease)
- Acantholytic acanthoma
- Verrucous affections caused by papillomavirus (HPV) (such as Verruca vulgaris, Verruca filiformis, Verruca plantaris, Verruca plana, Epidermodysplasia verruciformis, Condyloma accuminatum and/or Bowenoid papulosis of the genitalia)
- Inverted follicular keratosis
- Molluscum contagiosum
- Arsenical keratosis
- Large cell acanthoma
- Epidermolytic acanthoma
- Knuckle pads
- Keratoacanthoma
- Pale cell acanthoma
- Pale cell papulosis
- Pseudoepitheliomatous hyperplasia

In another preferred embodiment of the invention, said tumourous pathological condition of the skin is a dermatological disorder caused by Tumorous infiltration of the corium, preferably selected from one or more of the following:
- Urticaria pigmentosa
- Histiocytosis X
- Lymphatic leukemia B
- Metastatic infiltration of the dermis
- Paget's carcinoma of the breast
- Extramammary Paget's disease
- Epidermal spread of colonic adenocarcinoma

In another preferred embodiment of the present invention, said tumourous skin disorder is a malignant epidermal tumor, preferably selected from one or more of the following:
- Solar keratosis
- Actinic cheilitis
- Bowen's intraepidermal carcinoma
- Bowen's disease, pagetoid
- Queyrath's disease
- Vulval intraepithelial neoplasia (VIN III.)
- Cornu cutaneum
- Basalioma
- Squamous cell carcinoma
- Verrucous carcinoma
- Giant condylomata of Buschke-Loewenstein

In another preferred embodiment of the present invention, said tumourous skin disorder is an adnexal tumor, preferably a tumour of the hair follicle, such as selected from one or more of the following:
■ Hair follicle nevus
■ Trichofolliculoma
■ Trichoadenoma
■ Dilated pore of Winer
■ Pilar sheath acanthoma
■ Trichoepithelioma
■ Trichoblastoma
■ Tricholemmoma (such as Desmoplastic tricholemmoma)
■ Trichomatricom (pilomatrixom)
■ Folliculosebaceous hamartoma

Equally preferably, said adnexal tumor is a tumour of the sweat glands, such as selected from one or more of the following:
■ Eccrine nevus (such as Eccrine angiomatous hamartoma)
■ Cylindroma
■ Eccrine spiradenoma
■ Nodular hidradenoma (such as Cystic nodular hidradenoma)
■ Syringoma
■ Papillary eccrine adenoma
■ Apocrine nevus
■ Chondroid syringoma
■ Syringocystadenoma papilliferum
■ Hidradenoma papilliferum
■ Poroma (such as one or more of Eccrine poroma, Hidroacanthoma simplex, Dermal duct tumor, Poroma,malignant (porocarcinoma), Syringofibroadenoma, Intraepidermal epithelioma of Borst Jadassohn)
■ Microcystic adnexal carcinoma
■ Adenoid cystic carcinoma

Equally preferably, said adnexal tumor is a sebaceous tumor, such as selected from one or more of the following:
■ Ectopic sebaceous glands
■ Nevus sebaceus (of Jadassohn)
■ Sebaceous hyperplasia (such as Sebaceous hyperplasia of the penis)
■ Sebaceous adenoma
■ Sebaceoma, sebaceous epithelioma
■ Muir-Torre syndrome

In another preferred embodiment of the present invention, said tumourous skin disorder is a mesenchymal tumor, preferably one or more of the following:
■ Fibroma
■ Dermatofibroma (histiocytoma of the skin)
■ Atypical fibroxantoma
■ Dermatofibrosarcoma protuberans ■ Giant cell fibroblastoma
■ Skin tags
   ■ Fibroepithelial polyp
   ■ Supernumerary digit
   ■ Supranumerary nipple
   ■ Accessory tragus
   ■ Acral fibrokeratoma
   ■ Acquired periungal fibrokeratoma
   ■ Vestibular papules of the vulva
   ■ Focal dermal hypoplasia of Golz
■ Keloid
■ Lipoma
   ■ Common lipoma
   ■ Angiolipoma
   ■ Liposarcoma
■ Vascular tumors
   ■ Benign vascular tumors
      ■ Capillary hemangioma
      ■ Cavernous hemangioma
         ■ Sinusoidal hemangioma
         ■ Blue rubber bleb nevus syndrome
         ■ Nevus simplex
         ■ Nevus flammeus
         ■ Arteriovenous malformation
         ■ Venous malformation
         ■ Spindle cell hemangioma
         ■ Angiokeratoma
         ■ Granulation tissue
         ■ Intravascular hemangioma of Masson
         ■ Granuloma pyogenicum
         ■ Senile hemangiomas
         ■ Acquired tufted hemangioma
         ■ Microvenular hemangioma
         ■ Verrucous hemangioma
         ■ Targetoid hemosiderotic hemangioma
         ■ Nevus araneus
         ■ Acral arteriovenous hemangioma
         ■ Epitheloid hemangioma (angiolymphoid hyperplasia)
      ■ Vascular tumors of borderline malignancy
         ■ Epitheloid hemangioendothelioma
         ■ Retiform hemangioendothelioma
      ■ Malignant tumors of blood vessels
         ■ Kaposi's sarkoma
         ■ Angiosarcoma
   ■ Tumors of lymphatic vessels
      ■ Lymphangioma
      ■ Glomus tumor
      ■ Glomangioma
      ■ Tumors of the smooth muscle
         ■ Pilar leiomyomas
         ■ Angioleiomyoma
         ■ Genital leiomyomas
         ■ Leiomyosarcoma
      ■ Adenoma (angiofibroma) sebaceum Pringle
      ■ Histiocytic tumors and proliferations
         ■ Xanthoma
         ■ Juvenile xanthogranuloma
         ■ Necrobiotic xanthogranuloma
         ■ Benign cephalic histiocytosis
         ■ Giant cell tumor of the tendon sheath
      ■ Neurogenic tumors
         ■ Benign neurogenic tumors
            ■ Neurofibroma
            ■ Recklinghausen's disorder
            ■ Schwannoma (neurilemmoma)
            ■ Traumatic neuroma
            ■ Neurothekeoma
            ■ Granular cell tumor, Abrikosoff
      ■ Lymphomas
         ■ Pseudolymphoma, lymphocytoma
            ■ B cell pseudolymphoma
            ■ T cell pseudolymphoma
         ■ Cutaneous lymphoproliferative infiltrates other than lymphomas
            ■ Insect bite reaction
            ■ Sinus histiocytosis with massive lymphadenopathy Rosai-Dorfman
            ■ Leishmaniasis
            ■ Angioimmunoblastic lymphadenopathy
         ■ Skin lesions in nodal lymphomas
         ■ T cell lymphomas
            ■ Small plaque parapsoriasis
            ■ Large plaque parapsoriasis
            ■ Lymphomatoid papulosis
            ■ Mycosis fungoides
            ■ Sezary syndrome
            ■ Pagetoid reticulosis Worringer Kolopp
            ■ Syringolymphoid hyperplasia with alopecia
            ■ Pleomorphic small to medium-sized T cell lymphoma
            ■ Pleomorphic medium-sized and large T cell lymphoma (HTLV-1 +/-)
            ■ Anaplastic large cell lymphoma T
            ■ Adult T cell lymphoma
            ■ Granulomatous Slack Skin
            ■ Lymphomatoid granulomatosis (Liebow)
            ■ Juvenile granulomatous cutaneous T cell lymphoma
            ■ B cell rich T cell lymphoma
            ■ Subcutaneous panniculitis-like T cell lymphoma
            ■ CD8 positive T cell lymphoma
            ■ T cell receptor gamma/delta chain positive lymphoma
            ■ NK cell lymphoma (CD56+)
            ■ Angiocentric and angiodestructive lymphoma
            ■ T zone lymphoma, Lennert and Mohri
            ■ Intravascular T lymphoma
         ■ B cell lymphomas
            ■ Follicular lymphoma
               ■ Secondary skin infiltration in nodal follicular lymphoma
            ■ Marginal zone lymphoma
            ■ Immunocytoma
            ■ Mantle cell lymphoma
            ■ Large cell B lymphoma
            ■ Plasmocytoma
            ■ T cell rich B cell lymphoma
            ■ Intravascular B lymphoma
            ■ Lymphoblastic lymphoma B
      ■ Skin infiltrates in granulocytic malignancies
         ■ Acute myeloic leukemia
         ■ Chloroma

In another preferred embodiment of the present invention, said tumourous skin disorder is a melanocytic tumor, preferably selected from one or more of the following:
■ Melanocytic nevus
   ■ Common melanocytic nevi
      ■ Lentigo simplex
      ■ Junctional melanocytic nevus
      ■ Compound melanocytic nevus
      ■ Dermal nevus
      ■ Speckled lentiginous nevus, nevus spilus
   ■ Balloon cell nevus
   ■ Halo nevus
   ■ Recurrent melanocytic nevus
   ■ Giant melanocytic nevus
   ■ Nevus Spitz
      ■ Pigmented spindle cell nevus (Reed)
   ■ Blue nevus
      ■ Blue nevus of common type, of dendritic melanocytes
      ■ Cellular blue nevus
      ■ Special forms of blue nevi
      ■ Combined nevi
   ■ Melanocytic nevus of the conjunctiva
■ Melanoma
   ■ Lentigo maligna
   ■ Lentigo maligna melanoma
   ■ Intraepidermal melanoma (melanoma in situ)
   ■ Superficial spreading melanoma, SSM
      ■ Superficial spreading melanoma, vertical growth
   ■ Nodular melanoma
   ■ Acrolentiginous and mucosal melanoma
   ■ Melanoma ex nevo
   ■ Balloon cell melanoma
   ■ Melanoma metastases
Equally preferably, said tumourous skin disorder is a Merkel cell carcinoma.

In another preferred embodiment of the present invention, an ACE inhibitor and/or angiotensin II receptor antagonist is used for the preparation of a medicament for the treatment of a dermatological disorder caused by, or associated with, a cyst of the skin and/or subcutis. In one preferred embodiment of the invention, said cyst is a cyst with epithelial lining, preferably one or more of the following:
- Cysts with lining of epidermal type
- Epidermal (infundibular) cyst
- Epidermal cyst combined with HPV infection
- Epidermal verrucous cyst combined with HPV infection
- Epidermal inclusion cyst
- Hair matrix cyst
- Trichilemmal cyst
- Proliferating trichilemmal cyst
- Dermoid cyst
- Cystic teratoma
- Pigmented follicular cyst
- Steatocystoma multiplex
- Skin keratocyst
- Vellus hair cyst
- Giant comedo
- Milium
- Thymic cyst
- Bronchogenic cyst
- Branchial cleft cyst
- Cutaneous ciliated cyst of the lower limbs
- Vulval mucinous and ciliated cyst
- Endometriosis
- Median raphe cyst
- Hidrocystoma (such as Eccrinne hidrocystoma or Apocrinne hidrocystoma (and its papillary variant))

In another preferred embodiment of the present invention, said cyst of the skin and/or subcutis is a non-epithelial cyst or pseudo cyst, preferably one or more of the following:
- Ganglion, synovial cyst
- Digital mucous cyst
- Mucocele
- Pilonidal sinus
- Pseudocyst of the auricle
- Parasitic cyst

In another preferred embodiment of the present invention, said dermatological disorder is caused by, or associated with, a dermatovenereal disease. In one preferred embodiment of the invention, said dermatovenereal disease is preferably one or more of the following:
- Syphilis
- Gonorrhea
- Trichomonas vaginalis
- Pathology of the foreskin, phimosis, balanitis (such as Balanitis, such as Balanitis plasmocellularis Zoon and/or Phimosis, paraphimosis, frenulum breve)

### Administration

It is preferred that the medicament is administered in a concentration equivalent of from 0.01 mg/kg body weight to 100 mg/kg body weight, such as 0.1 mg/kg body weight to 90 mg/kg body weight, such as 0.5 mg/kg body weight to 75 mg/kg body weight such as 1 mg/kg body weight to 60 mg/kg body weight, such as 2 mg/kg-45mg/kg body weight, such as 5 mg/kg body weight to 30 mg/kg body weight, such as 10 mg/kg body weight to 15 mg/kg body weight, such as 10 mg/kg body weight to 12 mg/kg body weight. In another preferred embodiment, said ACE inhibitor or angiotensin II receptor antagonist is present in an amount between about 0.1 mg/kg body weight to 10 mg/kg body weight. It is further preferred that the compounds of the present invention in the medicament disclosed herein may be administered in combination with one or more other compounds, as described in the "combinations" section below.

In one preferred embodiment of the present invention, the individual is a postmenopausal, female human being. In another, equally preferred embodiment, said individual is a pre-menopausal, female human being. In another, equally preferred embodiment, said individual is a male human being.The compositions of the invention can be applied to the skin on an as-needed basis, for example, they can be applied to the skin in the morning and/or in the evening, and/or during the day. It is preferred that topical application be once a month to about 7 or 8 times daily, preferably from about 7 times a week to about 4 times a day, most preferably about twice a day. To maintain the beneficial effects of the medicament, it is preferred that said contacting is repeatedly performed over an extended period of time, preferably over the lifetime of the user, equally preferably for a period from 4 weeks to twenty years, more preferably from about 6 months to about five years. In another preferred embodiment of the present invention, the contacting is conducted at least once daily.

In one preferred embodiment of the present invention, said medicament further comprises a pharmaceutically-acceptable topical carrier.

### Preferred formulations of the cosmetic and pharmaceutical compositions disclosed herein

The cosmetic or pharmaceutical compositions according to the invention can be formulated in any form acceptable for their use in cosmetics and/or in pharmacy. More preferably, the compositions of the present invention are formulated in any suitable manner for application to an individual's skin. Preferably, the composition is in a form appropriate for topical application, more preferably suitable for the application to the face, hands, bust or body. It is preferred that said compositions are formulated as a lotion, face mask, skin patch, cream, ointment, water-based liquid, oil-based liquid, paste or sprayable liquid. More preferably, said composition is formulated as a cream or lotion.

In another preferred embodiment of the present invention, said formulation may contain ingredients such as absorbent particles (e.g. polymer beads or micelles) that provide sustained release of the compounds of the present invention to the skin. In another preferred embodiment, the formulations of the compositions of the present invention are hypo-allergenic, i.e. cause at the most a very low level of allergic reactions.

Compositions of the present invention can be directly applied, preferably to the skin, by any appropriate method, such as a spray bottle, a droplet bottle, a moisturized cotton ball or pad, suitable applicators such as paddles or strips, or by hands or fingers. In one preferred embodiment of the invention, compositions of the present invention may also be applied in a skin patch that incorporates cosmetic or pharmaceutical substances therein, such as those disclosed in French Patent Applications No. 2 512 651 and 2 538 247. In another, equally preferred embodiment of the present invention, compositions of the present invention may be applied in a skin "mask", preferably in the form of a gels or paste, such as those skin masks disclosed in European Patent Application No. 0 063 875, Austrian Patent No. 206 114 and US5026552.

Another preferred form for topical delivery of the compounds of the present invention is a hot compress comprising a woven or non-woven fibrous wrap impregnated with one or more compounds of the present invention. It is preferred that prior to treatment the impregnated fibrous wrap is immersed in warm water to at least partially solubilize the active component and is wrapped around the area to be treated. Another preferred form of topical delivery is film-forming materials loaded with the compositions of the present invention. Such film-forming materials are, for example, disclosed in U.S. Pat. No. 4,623,539, which is incorporated herein by reference. Said film-forming polymers may include certain anionic, cationic and neutral polymers.

The compositions may also be packaged in the form of an aerosol composition containing a propellent agent under pressure.

It is preferred that the compositions of the present invention are combined with a cosmetically or pharmaceutically or dermatologically acceptable carrier. The total amount of the carrier preferably ranges from about 10 to about 99.9%, preferably from about 50 to about 90%, optimally from about 70 to about 85% by weight of the formulation.

### Cosmetic or dermatological or pharmaceutical acceptable carrier

"Cosmetic or dermatological or pharmaceutical acceptable carrier", refers to a vehicle, for either cosmetic, dermatological or pharmaceutical use, which delivers the active components to their site of action and will not cause significant harm to the human or animal recipient. Any carrier selected for use in the therapeutic and cosmetic compositions should be pharmaceutically and/or cosmetically acceptable and appropriate for the form in which the composition will be used, e.g., cream, gel, milk, oil, lotion, face mask, skin patch, ointment, water-based liquid, oil-based liquid, paste, sprayable liquid and the like. Preferably, the carrier has an affinity for the skin, and/or is well tolerated and/or stable and/or it is used in an amount adequate to provide the desired consistency and ease of application.

The physiologically acceptable carrier in which the compounds according to the invention can be used, as well as the components thereof, their amount, the galenic form of the composition and its preparation mode, can be selected by the man of the art on the basis of his general knowledge and depending on the type of the desired composition. Those skilled in the art will appreciate that a wide variety of pharmaceutically or cosmeceutically-acceptable carriers may be employed according to the present invention. Examples of such carriers are described in U.S. Pat. No. 4,877,805 and EPA Pub. No. 0586106A1.

In one embodiment of the present invention, said carrier may be a simple combination of a buffered solution of propylene glycol, and an acrylate gelation agent, or any of a wide variety of known or commercially available formulations for e.g. creams or lotions. More than one type of carrier may be used. In a preferred embodiment of the present invention, said carrier has been shown to have beneficial effects for improving skin tone. Thus, for example, the carrier may be that disclosed according to U.S. Pat. No. 5,885,596, hereby incorporated by reference.

For applying onto the skin, the composition can have the form in particular of an aqueous or an oily solution; of a dispersion of the lotion or serum type, of emulsions of liquid or semi-liquid consistency of the milk type obtained through dispersion of a fatty phase into an aqueous phase (O/W) or reversely (W/O); of suspensions or emulsions of a soft consistency of the cream type or aqueous or anhydrous gel type; of microcapsules or microparticles; of vesicular dispersions of the ionic and/or non ionic type. When the composition is in an aqueous form, in particular in an aqueous dispersion, emulsion or solution, it can comprise an aqueous phase, which may comprise water, flower water and/or mineral water. Said aqueous phase can additionally comprise alcohols such as C₁-C₆ monoalcohols and/or polyols such as glycerol, butyleneglycol, isoprene glycol, propyleneglycol, polyethyleneglycol. Ointments and creams may be formulated with an aqueous or oil base with the addition of suitable thickening or gelling agents. Lotions may be formulated with an aqueous or oily base. Powders may be formulated with the aid of any suitable powder base, such as talc, lactose, starch and the like. Ointments, pastes, creams and gels of the present invention may contain excipients, such as paraffins, starch, tragacanth, cellulose derivatives, polyethylene glycols, silicones, talc and zinc oxide.

Generally, the carrier can be anhydrous or aqueous. It can thus comprise an aqueous phase and/or a fatty phase. Thus, in one preferred embodiment of the present invention, the compositions comprise a fatty phase, in particular made of fatty bodies liquid at 25 °C., such as oils from animal, vegetable, mineral or synthetic origin, either volatile or not, fatty bodies solid at 25 °C such as waxes from animal, vegetable, mineral or synthetic origin; of pasty fatty bodies; of gums; and the mixtures thereof. The volatile oils are generally oils having, at 25 °C, a saturating vapor tension at least equal to 0.5 millibar (50 Pa). Fatty phase components include, but are not restricted to: cyclic volatile silicones having 3 to 8 silicon atoms, preferably 4 to 6, cyclocopolymers of the dimethylsiloxane/methylalkylsiloxane type, linear volatile silicones with 2 to 9 silicon atoms, hydrocarbon volatile oils, such as isoparaffins and, more particularly, isododecane and fluorinated oils, poly(C₁-C₂₀)alkylsiloxanes and, more particularly, those with trimethylsilyl end groups, amongst which linear polydimethylsiloxanes and alkylmethylpolysiloxanes such as cetyldimethicone (CTFA name), silicones modified by aliphatic and/or aromatic groups, optionally fluorinated, or by functional groups such as hydroxyl, thiol and/or amine groups, phenylated silicone oils, oils from animal, vegetable or mineral origin, in particular animal or plants oils made of esters of fatty acids and polyols, in particular liquid triglycerids, for example sunflower, corn, soya, marrow, grape seed, sesame, hazelnut, apricot, almond, or avocado oils; fish oils, glycerol tricaprocaprylate, or plant or animal oils having the formula R₁COOR₂, where R₁ represents the residue of a superior fatty acid having 7 to 19 carbon atoms and R₂ represents a branched hydrocarbon chain having 3 to 20 carbon atoms, for example Purcellin oil; paraffin oil, liquid paraffin, perhydrosqualene, wheatgerm, calophyllum, sesame, macadamia, grape seed, colza, copra, arachis, palm, castor, jojoba, olive or cereal germ oils; fatty acid esters; alcohols; acetylglycerides; octanoates, decanoates or ricinoleates from alcohols or polyalcohols; fatty acid triglycerids; glycerids; fluorinated and perfluorinated oils; silicone gums; waxes from animal, vegetable, mineral or synthetic origin, such as microcrystalline waxes, paraffin, petrolatum, liquid paraffin, ozokerite, Montan wax; beewax, lanolin, and the derivatives thereof; Candelilla, Ouricury and Japan waxes, cocobutter, cork fibre or sugar cane waxes; hydrogenated oils solid at 25 °C, ozokerites, fatty esters and glycerides solid at 25 °C; polyethylene waxes and waxes obtained through Fischer-Tropsch synthesis; hydrogenated oils solid at 25 °C; lanolins; fatty esters solid at 25 °C; silicone waxes; fluorinated waxes.

Other suitable carriers for use with the present invention include, but are not limited to, water, mineral oil, ethylene glycol, propylene glycol, lanolin, glyceryl stearate, sorbitan stearate, isopropyl myristate, isopropyl palmitate, acetone, glycerol, phosphatidylcholine, sodium cholate, or ethanol.

When present, the amount of water in a composition may range anywhere from about 1 to about 99%, preferably from about 20 to about 90%, optimally between about 40 and 70% by weight.

The composition according to the invention can also comprise at least one co-emulsifier, which includes, but is not restricted to, oxyethylenated sorbitan monostearate, fatty alcohols such as stearyl alcohol or cetyl alcohol, or esters of fatty acids and polyols such as glyceryl stearate.

The compositions may also be combined with a skin penentration enhancer. The enhancers, helping to transport the active components through the normal intact skin, include, but are not limited to, liposomes, mixed lipid micelles, ethosomes, transfersomes, niosomes, ethanol, amides, ethers, glycols, hydrocarbon oils, sodium lauryl sulfate, oleic acid, hydroalcoholic solution, and soya phosphatidylcholine or their combinations. Other skin penetration enhancers includes use different pH values, co-solvents, surfactants, cyclodextrins, and iontophoresis. Said skin penetration enhancer is preferably in an amount ranging from 0.01 to 30% by weight based on the total weight of the composition. In one preferred embodiment, said skin penetration enhancer is a natural surfactants or an artificial surfactant such as isopropyl myristate.

Suitable solvents which can be used in the invention include lower alcohols, in particular, ethanol and isopropanol, and propylene glycol. Suitable hydrophilic gelling agents include carboxyvinyl polymers (carbomer), acrylic copolymers such as acrylate/alkylacrylate copolymers, polyacrylamides, polysaccharides such as hydroxypropylcellulose, natural gums and clays. Suitables lipophilic gelling agents include modified clays such as bentones, metal salts of fatty acids such as aluminum stearates, and hydrophobic silica, or alternatively ethylcellulose and polyethylene.

### Stabilization

It is preferred that the compositions of the present invention are stabilized. In general, stabilization methodologies and techniques that may be used in accordance with the present invention include any and all methods for the stabilization of chemical or biological material known to the art, including without limitation the addition of chemical agents, temperature modulation based methodologies; radiation based methodologies or combinations thereof. In preferred embodiments, small amounts of stabilizing chemical agents are mixed with the formulation comprising compositions of the present invention in order to achieve a stable preparation. These chemical agents preferably constitute less than approximately 10% (w/w), more preferably less than about 5% (w/w) and most preferably less than about 2.5% (w/w) of the formulation. Chemical agents that may be used in accordance with the present invention include inter alia preservative agents; acids; bases; salts; anti-oxidants; viscosity modifying agents; emulsifiers; gelling agents; and mixtures thereof.

In accordance with the present invention, oxidative reactions may be prevented by the addition of anti-oxidants to the compounds of the present invention, for example butylated hydroxytoluened (BHT); butylated hydroxyanisol (BHA); methyl hydroxybenzoate, propyl hydroxybenzoate and benzalkonium chloride, ascorbic acid (vitamin C), tocopherol, tocopherol acetate, phytic acid, citric acid, pro-vitamin A, and mixtures thereof. More preferably, BHA and/or BHT are used.

The physical stability of the formulation of the compositions of the present invention may be further enhanced by the addition of emulsifying agents. Any emulsifying agent may be used. Examples of suitable emulsifying agents include, but are not restricted to, Arlacel, such as Alacel 165; or Glucamate.

Preservatives and/or antimicrobial actives are also suitable for use in combination with the compounds of the present invention, such as all antibiotics, antimicrobial agents and antimicrobial peptides. Antibiotics that may be used include inter alia dermatologically acceptable salts of tetracylin and tetracyclin derivatives, gentamycin, kanamycin, streptomycin, neomycin, capreomycin, lineomycin, paromomycin, tobramycin, erythromycin, triclosan, octopirox, parachlorometa xylenol nystatin, tolnafiate, miconazole hydrochloride, chlorhexidine gluconate, chlorhexidin hydrochloride, methanamine hippurate, methanamine mandelate, minocycline hydrochloride, clindamycin, cloecin, b-lactam derivatives such as aminopenicillin and mixtures thereof. Preferred compounds for use with the present invention are chlorhexidin gluconate and tricolosan. Anti microbial agents that may be used in accordance with the present invention include for example benzoyl peroxide and salicylic acid. Antimicrobial peptides useful herein are for example magainin, nicin and cecropin.

Other preservatives suitable for use in combination with the compounds of the present invention include, but are not restricted to, sodium metabisulfite, Glydant Plus, Phenonip, methylparaben, Germall 115, Germaben II, phytic acid, sodium lauryl sulfate (SLS), methyl hydroxybenzoate, propyl hydroxybenzoate, benzalkonium chloride, sodium lauryl ether sulfate (SLES), and mixtures thereof. In preferred embodiments non-formaldehyde donors such as Neolone, Kathon and Euxyl are used. alkyl esters of para-hydroxybenzoic acid, hydantoin derivatives, propionate salts, and a variety of quaternary ammonium compounds. Chemists are familiar with appropriate preservatives and routinely choose them to satisfy the preservative challenge test and to provide product stability. Particularly preferred preservatives are methyl paraben, imidazolidinyl urea, sodium dehydroxyacetate, propyl paraben and benzyl alcohol. The preservatives should be selected having regard for the use of the composition and possible incompatibilities between the preservatives and other ingredients in the composition. Preservatives are preferably employed in amounts ranging from about 0.01 % to about 2% by weight of the composition.

### Viscosity modifiers

Compositions of the invention can also include viscosity modifiers, preferably in amounts from about 0.01 to about 10% by weight of the composition. Viscosity modifiers such as cetyl alcohol, glycerol, polyethylene glycol (PEG), PEG-stearate, or Keltrol may also be used to enhance the stability of the formulation. Thickeners which may enhance the stability include gelling agents such as cellulose and derivatives, Carbopol and derivatives, carob, carregeenans and derivatives, xanthane gum, sclerane gum, long chain alkanolamides, bentone and derivatives, Kaolin USP, Veegum Ultra, Green Clay, Bentonite NFBC, magnesium aluminum silicate (Veegum ®), guar gums (such as Jaguar HP-120 ®), xanthan gum, sodium carboxymethyl cellulose, hydroxyalkyl and alkyl celluloses, cross-linked acrylic acid polymers such as those sold by B. F. Goodrich under the Carbopol ® trademark, and mixtures thereof. As known to those skilled in the art, the precise amount of thickeners can vary depending upon the consistency and thickness of the composition which is desired.

### Combinations

In one embodiment of the present invention, the compositions of the present invention may be administered in combination with other compounds, which may have a therapeutic, cosmetic or otherwise beneficial effect. By "in combination", it is meant that said other compounds may administered before, concurrently with, or after, administration of the compositions of the present invention. Said other compounds may also be formulated with the ACE inhibitors and/angiotensin II antagonists of the present invention in the cosmetic compositions and/or medical compositions disclosed herein, in which case said other compounds, depending on their nature, may for example be introduced into the fatty phase, into the aqueous phase and/or into lipid spherules, of the compositions of the present invention. The nature and the amount of said other compounds can be selected one skilled in the art, based on common general knowledge, so as to obtain the desired presentation form for the composition. When said other compounds are added to the cosmetic and pharmaceutical compositions of the present invention, one skilled in the art could make sure to select suitable amounts of said other compounds, so that the advantageous properties of the composition according to the invention are not, or substantially not, altered by the contemplated addition.

Preferred compounds suitable for administration in combination with the compositions of the present invention comprise, but are not restricted to, hormones, plant and/or herbal extracts, moisturizers or humectants, emollients, fragrances, sunscreen actives, anti-wrinkle and/or anti-ageing actives, whitening and/or bleaching actives, sunless tanning actives, preservative and/or antimicrobial actives, anti-acne actives, anti-psoriasis actives, anti-eczema actives, anti-inflammatory actives, vitamin actives, proteins, peptides, amino acids, amino acid derivatives, insect repellants, fungicides, anti-viral agents, anti-cancer agents, anti-hemorrhoid compounds, anti-dandruff compounds, hair-growth stimulating compounds, hair-loss stimulating compounds, nucleic acids, chelating agents, pigments, lipids and/or inorganic salts.

In a preferred embodiment of the present invention, the compositions of the present invention are administered in combination with more than one other compounds, such as 2, 3, 4, 5, 6, 7, 8, 9, 10, 11, 12, 13, 14, 15, 16, 17, 18, 19, 20, 21, 22, 23, 24, 25, 26, 27, 28, 29 or 30 other compounds. In another preferred embodiment of the present invention, the compositions of the present invention are administered in combination with over 30 other compounds, such as 35-40, such as 40-45, such as 45-50, such as 50-100 other compounds.

### Hormones

Preferred examples of suitable other compounds comprise hormones, such as oestrogenic, progestative or androgenic hormones, including but not restricted to progesterone, testosterone, anhydrous oestradiol, broparestrol, oestrone, pregnenolone acetate, pregnenolone, 17-β-hydroxyprogesterone, testosterone propionate, androstenedione and androstanediols. Said hormones may be natural or synthetic.

### Plant and herbal extracts

Equally preferred other compounds suitable for use in combination with the compounds of the present invention include, but are not restricted to, plant or herbal extracts or chemically synthesised equivalents, such as extracts of Paraguay tea, Kola and Guarana, mate, marama bean, aloe vera; cryocytol; avocado; chamomile; echinacea; ginko biloba; ginseng; green tea; heather; jojoba; lavender; evening primrose oil, Marigold, Almond oil, safflower oil, jojoba oil, wheat germ oil, Horse-chestnut, Cucumber, Ivy, Bladderwort, Jodalga extract, lemon grass; licorice; mallow; oats; peppermint; St. John's wort; willow; wintergreen; wheat wild yam; Ubiquinone Q10, Retinoids, Alpha hydroxy acids (AHAs), Antocopherol and marine extracts, such as seaweed extract.

### Moisturizers and humectants

Equally preferred compounds suitable for use in combination with the compounds of the present invention include, but are not limited to, one or more moisturizers. As used herein a "moisturizer" is an ingredient which promotes the retention of water to the surface area of the human body, including hair and skin. The term moisturizer as used herein includes both components which deliver water to the skin, also commonly referred to in the art as "humectant", and components which prevent the loss of water from the skin, also commonly referred to in the art as "occlusive". If present in the compositions of the present invention, said moisturizer will generally comprise from about 0.1% (w/v) to about 99% (w/v), more preferably from about 0.5% (w/v) to about 50% (w/v), and most preferably from about 1% (w/v) to about 40% (w/v) of the final composition.

Although the ingredients mentioned herein are generally defined as moisturizers they may also possess other properties such as emolliency or other conditioning properties. Moisturizers suitable for use in combination with the compounds of the present invention include, but are not limited to, polyhydroxy alcohols, including butylene glycol, hexylene glycol, propylene glycol, sorbitol and the like; lactic acid and lactate salts, such as sodium or ammonium salts; C₃ and C₆ diols and triols including hexylene glycol, 1,4 dihydroxyhexane, 1,2,6-hexane triol; aloe vera in any of its forms, for example aloe vera gel; sugars and starches; sugar and starch derivatives, for example alkoxylated glucose; hyaluronic acid; lactamide monoethanolamine; acetamide monoethanolamine; glycolic acid; alpha and beta hydroxy acids (e.g. lactic, glycolic salicylic acid); glycerine; pantheol; urea; vaseline; natural oils; oils and waxes (see: the emollients section herein) and mixtures thereof.

Humectants of the polyhydric alcohol-type may also be used in combination with the compositions of this invention. Possible roles of the humectant may be to aid in increasing the effectiveness of an emollient, reduce scaling, stimulate removal of built-up scale and improve skin feel. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably glycerol.

For improved lubricity, there may also be included one or more silicone oils or fluids which may be selected from a dimethyl polysiloxane, a methylphenyl polysiloxane and an alcohol-soluble silicone glycol copolymer. Preferred siloxanes include dimethyl polysiloxane (CTFA name: dimethicone), a polysiloxane end-blocked with trimethyl units and polydimethylcyclosiloxane, (CTFA name: cyclomethicone). The preferred siloxanes exhibit a viscosity from about 2 to 50 centistokes at 25 °C.

### Emollients

Equally preferred compounds suitable for use in combination with the compounds of the present invention include, but are not limited to, one or more emollients. Emollients may be used, for example, to add or replace lipids and natural oils to the surface area of the human body. The term emollient as used herein is intended to include conventional lipids (for example, oils, waxes, lipids and other water insoluble components) and polar lipids (lipids which have been modified in order to increase water solubility typically through esterfication of a lipid to a hydrophylic moiety for example hydroxy groups, carbonyl groups and the like). Preferred emollients suitable for use in combination with the compounds of the present invention include, but are not limited to, those selected from the group consisting of natural oils and preferably plant-derived and essential oils, esters, silicone oils, polyunsaturated fatty acids (PUFAs), lanoline and its derivatives and petrochemicals.

Natural oils which may be used in combination with the present invention include, but are not restricted to, those obtained from sesame; soybean; apricot kernel; palm; peanut; safflower; coconut; olive; cocoa butter; palm kernel; shea butter; sunflower; almond; avocado; borage; carnauba; hazel nut; castor; cotton seed; evening primrose; orange roughy; rapeseed; rice bran; walnut; wheat germ; peach kernel; babassu; mango seed; black current seed; jojoba; macademia nut; sea buckthorn; sasquana; tsubaki; mallow; meadowfoam seed; coffee; emu; mink; grape seed; thistle; tea tree; pumpkin seed; kukui nut; and mixtures thereof. Esters which may be used in combination with the present invention include, but are not restricted to, C₈ -C₃₀ alklyl esters of C₈ -C₃₀ carboxylic acids; C₁ -C₆ diol monoesters and diesters of C₈ -C₃₀ carboxylic acids; C₁₀ -C₂₀ alcohol monosorbitan esters, C₁₀ -C₂₀ alcohol sorbitan di- and tri-esters; C₁₀ -C₂₀ alcohol sucrose mono-, di-, and tri-esters and C₁₀ -C₂₀ fatty alcohol esters of C₂ -C₆ 2-hydroxy acids and mixtures thereof. Examples of these materials include isopropyl palmitate; isopropyl myristate; isopropyl isononate; C₁₂ /C₁₄ benzoate ester (also known as Finesolve); sorbitan palmitate, sorbitan oleate; sucrose palmitate; sucrose oleate; isostearyl lactate; sorbitan laurate; lauryl pyrrolidone carboxylic acid; panthenyl triacetate; and mixtures thereof.

Other preferred emollients include silicone oils, including non-volatile and volatile silicones. Examples of preferred silicone oils suitable for use in combination with the compounds of the present invention include, but are not limited to, dimethicone; cyclomethycone; dimethycone-copolyol; aminofunctional silicones; phenyl modified silicones; alkyl modified silicones; dimethyl and diethyl polysiloxane; mixed C₁ -C₃₀ alkyl polysiloxane; and mixtures thereof. Equally preferred silicones are described in U.S. Pat. No. 5,011,681 to Ciotti et al., incorporated by reference herein. Equally preferred emolliants suitable for use in combination with the compounds of the present invention include, but are not limited to, lanoline and lanoline derivatives for example lanoline esters. Petrochemicals suitable for use as emolliants in combination with the compounds of the present invention include, but are not limited to, mineral oil; petrolatum; isohexdecane; permethyl 101; isododecanol; C₁₁ -C₁₂ Isoparrafin, also known as Isopar H.

Waxes suitable for use in combination with the compounds of the present invention include, but are not limited to, animal waxes such as beeswax; plant waxes such as carnauba wax, candelilla wax, ouricurry wax, Japan wax or waxes from cork fibres or sugar cane, mineral waxes, for example paraffin wax, lignite wax, microcrystalline waxes or ozokerites and synthetic waxes.

Other emollients suitable for use in combination with the compositions of the present invention include, but are not restricted to, hydrocarbon oils and waxes, such as mineral oil, petrolatum, paraffin, ceresin, ozokerite, microcrystalline wax, polyethylene, and perhydrosqualene; triglyceride esters such as vegetable and animal fats and oils, such as castor oil, cocoa butter, safflower oil, cottonseed oil, corn oil, cod liver oil, almond oil, avocado oil, sesame oil, squalene, and maleated soybean oil; acetoglyceride esters, such as acetylated monoglycerides; ethoxylated glycerides, such as ethoxylated glyceryl monostedrate; alkyl esters of fatty acids having 10 to 22 carbon atoms, such as methyl, isopropyl, and butyl esters of fatty acids, such as hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate; alkenyl esters of fatty acids having 10 to 22 carbon atoms, such as oleyl myristate, oleyl stearate, and oleyl oleate; fatty acids having 10 to 22 carbon atoms, such as pelargonic, lauric, myristic, palmitic, stearic, isostearic, hydroxystearic, oleic, linoleic, ricinoleic, arachidic, behenic, and erucic acids; fatty alcohols having 10 to 22 carbon atoms, such as lauryl, myristyl, cetyl, hexadecyl, stearyl, isostearyl, hydroxystearyl, oleyl, ricinoleyl, behenyl, erucyl, and 2-octyl dodecanyl alcohols; fatty alcohol ethers, such as ethoxylated fatty alcohols of 10 to 22 carbon atoms, such as the lauryl, cetyl, stearyl, isostearyl, oleyl, and cholesterol alcohols, having attached thereto from 1 to 50 ethylene oxide groups or 1 to 50 propylene oxide groups; ether-esters such as fatty acid esters of ethoxylated fatty alcohols; lanolin and derivatives, such as lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases; polyhydric alcohol esters such as ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 mono- oleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid esters, sorbitan fatty acid esters, and polyoxy-ethylene sorbitan fatty acid esters; wax esters such as beeswax, spermaceti, myristyl myristate, stearyl stearate; beeswax derivatives, such as polyoxyethylene sorbitol beeswax; vegetable waxes including carnauba and candelilla waxes; phospholipids such as lecithin and derivatives; sterols, cholesterol and cholesterol fatty acid esters; amides such as fatty acid amides, ethoxylated fatty acid amides and solid fatty acid alkanolamides. When formulated with the compositions of the present invention, emollients preferably range from about 0.5 to about 80% by weight of the total composition. Preferably the amounts of these emollients will range from about 1 to about 25%, optimally between about 5 and 15% by weight. Equally preferably, said emollients typically comprise between from about 0.0 1 % to about 25%, preferably from about 0.05% to about 15% and more preferably from about 0.1 % to about 10% w/v of the total formulation.

It is noted that although the ingredients mentioned herein are generally defined as emollients they may also possess other properties such as moisturization or other advantageous properties.

### Fragrances

Equally preferred compounds suitable for use in combination with the compounds of the present invention include, but are not limited to, fragrances, preferably one or more fragrances without inherent adverse effects. If comprised in the pharmaceutical or cosmetic compositions of the present invention, said fragrance preferably comprises between about 0.000 1 % (v/v) and about 25% (v/v) of the final composition, more preferably between about 0.001 % (v/v) and 10% (v/v) and most preferably between 0.01 % (v/v) and 5% (v/v) of the final composition. For the purpose of the present application the term "fragrance" is meant to encompass any component reacting with the human olfactory sites and imparting a pleasurable odor, essence or scent.

Fragrances that may be used in accordance with the present invention include any synthetic as well as natural fragrance and mixtures thereof. Typically a multiplicity of fragrances are used to achieve the desired effect. Those of skill in the art further recognize the terms "top note" (i.e. fragrances having a high vapor pressure), "middle note" (i.e. fragrance having a medium vapor pressure) and "base note" (i.e. fragrances having a low vapor pressure). Recognizing that categorization within these classes may depend to some extent on the fragrance formulator, the fragrances used in combination with the present invention may comprise any top note, middle note and base note fragrance.

A further way of classifying fragrances is in accordance with generally recognized scents they produce. Descriptions used by those skilled in the art of fragrances are inter alia "rose", "floral", "green", "citrus", "spicy", "honey", "musk", "herbal", "jasmin", "lilac", "lily of the valley", "orange", "peach", "oriental", "watermelon" "chypre" and "lemon", "woody", "fruity" all of which fragrances thus classified may used in combination with the present invention. Preferred fragrances suitable for use in combination with the compounds of the present invention include, but are not limited to, linear and cyclic alkenes (i.e. terpenes); primary, secondary and tertiary alcohols; ethers; esters; ketones; nitrites; and saturated and unsaturated aldehydes; or mixtures thereof.

Equally preferred fragrances suitable for use in combination with the present invention include synthetic fragrances, such as one or more of acetanisole; acetophenone; acetyl cedrene; methyl nonyl acetaldehyde; musk anbrette; heliotropin; citronellol; sandella; methoxycitranellal; hydroxycitranellal; phenyl ethyl acetate; phenylethylisobutarate; gamma methyl ionone; geraniol; anethole; benzaldehyde; benzyl acetate; benzyl salicate; linalool; cinnamic alcohol; phenyl acetaldehyde; amyl cinnamic aldehyde; caphore; p-tertiairy butyl cyclohexyl acetate; citral; cinnamyl acetate; citral diethyl acetal; coumarin; ethylene brasslate; eugenol; 1-menthol; and vanillin.

Equally preferred fragrances suitable for use in combination with the present invention include natural fragrances, such as one or more of lavandin; heliotropin; sandlewood oil; oak moss; pathouly; ambergris tincture; ambrette seed absolute; angelic root oil; bergamont oil; benzoin Siam resin; buchu leaf oil; cassia oil; cedarwood oil; cassia oil; castoreum; civet absolute; chamomile oil; geranium oil; lemon oil; lavender oil and Ylang Ylang oil.

Equally preferred fragrances suitable for use in combination with the present invention include all the fragrances disclosed in "Perfume and Flavor Chemicals", Vols. I and II; Steffen Arctander Allured Pub. Co. (1994) and "Perfumes: Art, Science and Technology"; Muller, P. M. and Lamparsky, D., Blackie Academic and Professional (1994) both incorporated herein by reference.

### Sunscreen actives

Equally preferred compounds suitable for use in combination with the present invention include one or more sunscreen active. The term "sunscreen" is used to denote ultraviolet ray-blocking compounds inhibiting absorption within the wavelength region between 290 and 420 nm. These compounds may either be organic or inorganic. Typical inorganic sunscreens include titanium dioxide, zinc oxide, iron oxide and combinations thereof. Most preferred is titanium dioxide, especially having an average particle size no higher than 700 nm, preferably no higher than 200 nm, optimally less than 35 nm. Organic sunscreens may be classified into five groups based upon their chemical structures: para-amino benzoates; salicylates; cinnamates; benzophenones; coumarins; azoles and miscellaneous chemicals including menthyl anthralinate. Also polymeric particles may be useful such as polyethylene and polyamides. If incorporated into the compositions of the present invention, said organic sunscreen compounds preferably range in amount from about 0.1 to 25%, optimally from about 1 to 15%, most preferably from about 5 to 10% by weight. A wide variety of sunscreen actives are useful herein.

The exact amount and type of sunscreen that is used depends on the level of photoprotection that is desired. Generally, any agent offering protection against ultraviolet radiation by absorbing, scattering or reflecting the ultraviolet radiation may be used herein. The sunscreen agents may offer protection against one or more of the following forms of sunlight radiation UVA, UVB, UVC, visible light and infrared radiation. Generally the sunprotection factor (SPF) in the final formulation varies between 2 and 30, although products with SPFs up to 100 may be formulated. The sunscreen used herein may offer chemical or physical photoprotection. UVA and UVB blocking agents, such as those disclosed according to the U.S. Pat. No. 6,130,254 patent, may be included to provide a composition effective at preventing, minimizing or avoiding photoaging.

Equally preferred sunscreens suitable for use in combination with the compounds of the present invention include those selected from the group comprising amino benzoic acid and derivatives, such as para-amino benzoic acid (PABA), glyceryl-PABA (Lisadimate), Padimate O, Roxadimate; anthrinalates, including methylanthrynilate; benzophenones, including dioxybenzone, oxybenzone and sulisobenzone, 3-benzophenone (Uvinul M40) 4-N,N-dimethylaminobenzoic acid ester with 2,4-dihydroxybenzophenone; camphor derivatives including 3-(4-methylbenzylidene) camphor, 3-benzylidene camphor; cinnamates including DEA-p-methoxycinnamate, ethyl-hexyl p-methoxy cinnamate, octocrylene, octyl methoxy cinnamate (Parasol MCX); dibenzoyl methanes including butylmethoxydibenzoylmethane (Parsol 1789), salicylates including, homomenthyl salicylate, octyl salicylate, trolamine methyl salicylate; metal oxides including titanium dioxide, zinc oxide and iron oxide; 2-phenylbenzimidazole-5-sulfonic acid; 4,4-methoxy-t-butyldibenzoylmethane; and mixtures thereof.

Further non-limiting examples of sunscreens useful in combination with the present invention are described in U.S. Pat. No. 5,087,445 to Haffey et al., U.S. Pat. No. 5,073,372 to Turner et al. and U.S. Pat. No. 5,160,731 to Sabatelli et al., all of which are incorporated herein by reference in their entirety.

### Anti-wrinkle and anti-ageing actives

Equally preferred compounds suitable for use in combination with the present invention include anti-wrinkle and anti-aging actives. These agents include without limitation hydroxy acids including C₂ -C₃₀ alpha-hydroxy acids such as glycolic acid, lactic acid, 2-hydroxy butanoic acid, malic acid, citric acid tartaric acid, decorin-synthesis enhancers, retinoids which include retinol and its esters, retinal, retinoic acid and its derivatives, retinoids, and in particular those described in documents FR 2,570,377, EP 0 199 636, EP 0 325 540 and EP 0 402 072, .alpha.-hydroxy acids such as glycolic, lactic, malic, citric, tartaric or mandelic acid), .beta.-hydroxy acids such as salicylic acid and its derivatives, in particular its alkyl derivatives, .alpha.-keto acids, .beta.-keto acids, peroxides such as benzoyl peroxide, vitamins, in particular vitamins E and F. anti-free-radical active agents such as superoxide dismutase, selenium, zinc, beta-carotenes, tensioning polymers of natural or synthetic origin, collagen-synthesis enhancers, matrix metalloproteinases (MMP) inhibitors, antioxidants, collagen modulators, alpha-hydroxyethanoic acid, hydroxycaprylic acid and the like; alpha-hydroxycarboxylic acids or salts thereof, beta-hydroxycarboxylic acid or salts thereof, ceramides, polyhydroxy acids including gluconolactone (G4), gamma-linolenic acid, fruit acids, sugar cane extract and glycomer in cross-linked alpha nutrium; skin peel agents such as phenol, phytic acid and acetic acid.

### Whitening and/or bleaching actives

Equally preferred compounds suitable for use in combination with the compounds of the present invention include whitening and/or bleaching actives, which include hydroquinone and derivatives, kojic acid, lactic acid, niacinamide, ascorbyl acid and derivatives such as magnesium ascorbyl phosphate, arbutin, and licorice root. If incorporated directly into the cosmetic or pharmaceutical compositions of the present invention, said whitening or bleaching active is preferably present in an amount ranging from 0.001 to 10% by weight.

### Sunless tanning actives

Equally preferred compounds suitable for use in combination with the compounds of the present invention include sunless tanning actives, such as dihydroxyacetone (DHA); glyceryl aldehyde; tyrosine and tyrosine derivatives such as malyltyrosine, tyrosine glucosinate, and ethyl tyrosine; phospho-DOPA, indoles and derivatives; and mixtures thereof, possibly in combination with amines and/or amino acids. If incorporated directly into the cosmetic or pharmaceutical compositions of the present invention, said sunless tanning active is preferably present in an amount ranging from 1 % to 30% by weight.

### Anti-acne actives

Equally preferred compounds suitable for use in combination with the compounds of the present invention include one or more anti-acne actives, including, but not restricted to, keratolytic agents including lactic acid, pyruvic acid, salicylic acids, urea and N-acetylcysteine; retinoids, and retinoid analogs such as tretinoin, cis and trans retinoic acid, retinol and retinol palmitate, isotretinoin-13-cis-retinoic acid; antibiotics and antimicrobial agents such as tetracycline, erythromycin, minocycline, clindamycin, trimethoprim-sulphamethazole and anti-microbial peptides (nicin, for example); steroids, such as hydrocortisone; gamma-linolenic acid and mixtures thereof. Further anti-acne actives that may be used include without limitation benzoyl peroxide; salicylic acid, alpha and beta hydroxy acids; sulfacteamide and sulfur and derivatives and mixtures thereof. Preferably used herein are salicylic acid, benzoyl peroxide and retinoids. If incorporated directly into the cosmetic or pharmaceutical compositions of the present invention, said anti-acne active is preferably present in an amount ranging 0.1 to 30% by weight of the composition.

### Anti-psoriasis actives

Equally preferred compounds suitable for use in combination with the compounds of the present invention include one or more anti-psoriasis actives, including but not restricted to salicylic acid; mometasone furoate; steroids including corticosteroids such as cortisone and oluxclobetasol propionate; 5-fluorouracil; epinephrine; anthralin; vitamin D3 analogs, such as calcipotriene; methotrexate; masprocol; trimethaxate gluconate; retinoids; cyclosporin; paclitaxel; 5-amino levulinic acid; bergasol; tin-ethyl etio purpurin; benzoporphyrin derivatives; antibodies, such as ABX-IL8 antibody, CD11a monoclonal antibody and ICM3 monoclonal antibody; enzyme inhibitors, including tryptase inhibitor and phospholipase A-2 inhibitors; angiogenesis blocking agents; T-cell blocking agents and mixtures thereof.

### Anti-eczema actives

Equally preferred compounds suitable for use in combination with the compounds of the present invention include one or more of anti-eczema actives including, but not restricted to, urea, evening primrose oil, plant extracts, hydrocortisone, an immunomodulator, tar and/or fatty acids obtained from banana.

### Anesthetic actives

Equally preferred compounds suitable for use in combination with the compounds of the present invention include, but are not restricted to, one or more anesthetic actives, such as tetracaine, lidocaine, editocaine, bupivacaine or pramoxine.

### Anti-inflammatory actives

Equally preferred compounds suitable for use in combination with the compounds of the present invention include one or more anti-inflammatory actives, such as steroidal actives such as hydrocortisone, or non-steroidal actives including propionic derivatives, acetic acid derivatives, biphenylcarboxylic acid derivatives, fenamic acid derivatives, and oxicams; or acetominaphen, oxaprozin, pranoprofen, benoxaprofen, bucloxic acid or elocon.

### Vitamin actives

Equally preferred compounds suitable for use in combination with the compounds of the present invention include one or more of vitamin actives, including but not restricted to vitamin A and derivatives, including retinoic acid, retinyl aldehyde, retin A, retinyl palmitate, adapalene, and beta-carotene; vitamin B (panthenol, provitamin B5, panthenic acid, vitamin B complex factor); vitamin C (ascorbic acid and salts thereof) and derivatives such as ascorbyl palmitate; vitamin D including calcipotriene (a vitamin D3 analog) vitamin E including its individual constituents alpha-, beta-, gamma-, delta-tocopherol and cotrienols and mixtures thereof and vitamin E derivatives including vitamin E palmitate, vitamin E linolate and vitamin E acetate; vitamin K and derivatives; vitamin Q (ubiquinone) and mixtures thereof.

### Proteins and peptides

Equally preferred compounds suitable for use in combination with the compounds of the present invention include one or more protein or peptide. Proteins and/or peptides may be formulated in any desired manner for combination with the compounds of the present invention, however in one preferred embodiment, said proteins and/or peptides are recombinant. Proteins and/or peptides which may be used in combination with the compounds of the present invention include, but are not restricted to, enzymes such as proteases (e.g. bromelain, papain, collagenase, elastase), lipases (e.g. phospholipase C), esterases, glucosidases, exfoliating enzymes; antibodies and antibody derived actives, such monoclonal antibodies, polyclonal antibodies, single chain antibodies and the like; reductases; oxidases; peptide hormones; natural structural skin proteins, such as elastin, collagen, reticulin and the like; growth factors such as platelet derived growth factor (PDGF) and epidermis derived growth factor (EGF); anti-oxidants such as superoxide dismutase, catalase and glutathione; free-radical scavenging proteins; DNA-repair enzymes, for example T4 endonuclease 5 and P53; antimicrobial peptides, such as magainin and cecropin; a milk protein; a silk protein or peptide; and any active fragments or derivatives of the above-mentioned proteins and peptides.

### Other preferred compounds

Equally preferred compounds suitable for use in combination with the compounds of the present invention include one or more of: an amino acid and amino acid derivative; an insect repellant; a fungicide (such as ketoconazole); an anti-viral agent (such as acyclovir); an anti-cancer agent; an anti-hemorrhoid compound; an anti-dandruff compound; a hair-growth stimulating compound; a hair loss stimulating compound; a nucleic acid (which may be natural or non-natural); an anti-wart agent (such as podophyllotoxin); chelating agents (capable of binding metal ions) such as tartaric acid, EDTA, citric acid, alkali metal citrates, pyrophosphate salts or anionic polymeric polycarboxylates; pigments, which may be white or coloured, inorganic or organic and/or paerlescent.

Preferred pigments comprise, but are not restricted to, titanium dioxide, zinc oxide, zirconium dioxide, black, yellow, red and brown iron oxides, cerium dioxide, chromium oxide, ferric blue, carbon black, barium, strontium, calcium and aluminum lakes and mica coated with titanium oxide or with bismuth oxide.

Inorganic salts that may be used in combination with the compounds of the present invention include without limitation aluminum zirconium chloride; aluminum chlorohydroxide; zinc oxide; talc; borax; alum; ammonium acetate. These salts are particularly useful in preparing antiperspirants and deodorants.

### Other therapeutic or cosmetic methods

It is further envisaged that the compositions of the present invention may be administered in combination with other therapeutic or cosmetic methods, such as systemic therapies, such as oral administration of retinoids or vitamin C. Compositions of the present invention may also be administered in combination with chemical peels, for example using AHAs and BHAs or Trichloroacetic Acid (TCA), or exfoliants such as naturally occuring fruit acids, or mechanical facial scrubs. Equally preferably, the compositions of the present invention may be administered in combination with surgical procedures such as a face-lift, cosmetic facial remodelling or non-cosmetic facial remodelling. Equally preferably, compositions of the present invention may be administered in combination with injections of wrinkle-reducing/anti-ageing compounds such as hyaluronic acid, botox or collagen.

### Embodiments

The invention is exemplified by the following embodiments:
1. Use of an ACE inhibitor or pharmaceutically acceptable salt thereof and/or angiotensin II receptor antagonist or pharmaceutically acceptable salt thereof for the preparation of a medicament for the treatment of a dermatological disorder.
2. The use according to embodiment 1, wherein said dermatological disorder is associated with inflammation.
3. The use according to embodiment 2, wherein said disorder is dermatitis.
4. The use according to embodiment 2, wherein said disorder is eczema.
5. The use according to embodiment 2, wherein said disorder is psoriasis.
6. The use according to embodiment 2, wherein said disorder is a non-tumourous pathological condition of the skin.
7. The use according to embodiment 6, wherein said non-tumourous pathological condition of the skin is a disease of the epidermis, such as selected from Darier's disease or Pityriasis rubra pilaris.
8. The use according to embodiment 6, wherein said disorder is a blistering skin disorder.
9. The use according to embodiment 6, wherein said disorder is a a dermatological disorder caused by rubbing the skin and/or scratching the skin and/or pressure on the skin.
10. The use according to embodiment 6, wherein said disorder is a dermatological disorder caused by infiltration of the upper and/or deep corium.
11. The use according to embodiment 6, wherein said disorder is a dermatological disorder caused by one or more vasculitides.
12. The use according to embodiment 6, wherein said disorder is a dermatological disorder caused by one or more panniculitides
13. The use according to embodiment 6, wherein said disorder is a dermatological disorder caused by bacterial disease.
14. The use according to embodiment 6, wherein said disorder is a dermatological disorder caused by myotic disease.
15. The use according to embodiment 6, wherein said disorder is a dermatological disorder caused by parasitic, insect-borne or viral disease.
16. The use according to embodiment 6, wherein said disorder is a dermatological disorder caused by a disease of the hair follicles.
17. The use according to embodiment 6, wherein said disorder is a dermatological disorder caused by a disease of the nails.
18. The use according to embodiment 6, wherein said disorder is a dermatological disorder caused by a disease of the sweat glands.
19. The use according to embodiment 6, wherein said disorder is a dermatological disorder caused by an exogenic alteration of the skin.
20. The use according to embodiment 6, wherein said disorder is a dermatological disorder caused by a metabolic and/or nutrition deficiency or imbalance.
21. The use according to embodiment 6, wherein said disorder is a dermatological disorder caused by a drug reaction.
22. The use according to embodiment 6, wherein said disorder is a dermatological disorder caused by Organoid nevi and/or malformations of the skin.
23. The use according to embodiment 6, wherein said disorder is a dermatovenereal disease.
24. The use according to embodiment 1, wherein said dermatological disease is associated with excessive or undesirable fibroproliferative activation.
25. The use according to embodiment 24, wherein said dermatological disease is a dermatological disorder caused by disorders of melanin pigmentation.
26. The use according to embodiment 24, wherein said dermatological disease is a benign epidermal tumor.
27. The use according to embodiment 24, wherein said dermatological disease is a dermatological disorder caused by Tumorous infiltration of the corium.
28. The use according to embodiment 24, wherein said dermatological disease is a malignant epidermal tumor.
29. The use according to embodiment 24, wherein said dermatological disease is an adnexal tumor, preferably a tumour of the hair follicle.
30. The use according to embodiment 24, wherein said dermatological disease is a tumour of the sweat glands.
31. The use according to embodiment 24, wherein said dermatological disease is a sebaceous tumor.
32. The use according to embodiment 24, wherein said dermatological disease is a mesenchymal tumor.
33. The use according to embodiment 24, wherein said dermatological disease is a melanocytic tumor.
34. The use according to embodiment 24, wherein said dermatological disease is a Merkel cell carcinoma.
35. The use according to embodiment 24, wherein said dermatological disease is a cyst of the skin and/or subcutis.
36. The use according to embodiment 24, wherein said dermatological disease is Ichthyosis.
37. The use according to embodiment 36, wherein said Ichthyosis is selected from Ichthyosis vulgaris, Ichthyosis lamellaris, X-linked ichthyosis, Epidermolytic hyperkeratosis and/or Ichthyosis acquisita.
38. The use according to embodiment 24, wherein said dermatological disease is Psoriasis
39. The use according to embodiment 38, wherein said Psoriasis is selected from the group consisting of: psoriatic erytroderma, Palmoplantar psoriasis, palmoplantar pustulosis, generalized pustular psoriasis of Zumbusch and/or Lingua geographica.
40. The use according to embodiment 24, wherein said dermatological disease is Porokeratosis.
41. The use according to embodiment 24, wherein said dermatological disease is a dermatological disorder caused by perivascular infiltration of the upper dermis.
42. The use according to embodiment 24, wherein said dermatological disease is a dermatological disorder caused by diffuse infiltration of the upper dermis.
43. The use according to embodiment 24, wherein said dermatological disease is a dermatological disorder caused by granulomatous processes of the deeper corium.
44. The use according to embodiment 24, wherein said dermatological disease is a dermatological disorder caused by a connective tissue disorder.
45. The use according to embodiment 24, wherein said dermatological disease is a disorder of the elastic fibres
46. The use according to embodiment 24, wherein said dermatological disease is a dermatological disorder caused by deposition of foreign material in the dermis.
47. The use according to any of embodiments 1-46, wherein said ACE inhibitor and/or angiotensin II receptor antagonist is selected from Alacepril, Delapril Benazepril, Cilazapril,Captopril, Enlapril, Fosinopril, Lisinopril, Moexipril, Perindopril, Ramipril, Pentopril, Zofenopril, Quinapril, Trandolapril, Imidapril, Isradipin, perindopril, spirapril, temocapril, Enalapril, losartan (Cozaar), valsartan (Diovan), irbesartan (Avapro), candesartan (Atacand), telmisartan (Micardis), eprosartan, tasosartan, zolarsartan, Zofenapril, Isradipin and Candesartancilexetil, or a pharmaceutically acceptable salt thereof.
48. Use according to any of embodiments 1-46, wherein said ACE inhibitor and/or angiotensin II receptor antagonist is selected from Alacepril, Delapril , Cilazapril, Benazepril, Captopril, Enlapril, Fosinopril, Lisinopril, Moexipril, Perindopril, Ramipril, Pentopril, Zofenopril, Quinapril, Trandolapril, Imidapril, Isradipin, perindopril, spirapril, temocapril, Enalapril, Zofenapril, or a pharmaceutically acceptable salt thereof.
49. Use according to any of embodiments 1-46, wherein said ACE inhibitor or angiotensin II receptor antagonist is selected from losartan (Cozaar), valsartan (Diovan), irbesartan (Avapro), candesartan (Atacand), telmisartan (Micardis), eprosartan, tasosartan, zolarsartan, Isradipin, Candesartancilexetil and/or olmesartan medoxomil, or a pharmaceutically acceptable salt thereof.
50. Use according to any of embodiments 1-46, wherein said ACE inhibitor or angiotensin li receptor antagonist is selected from the group consisting of:
   quinaprilat, trandolaprilat, moexiprilat, fosinoprilat, fosinopril, benazeprilat, enalaprilat or ramaprilat.
51. Use according to any of embodiments 1-46, wherein said ACE inhibitor or angiotensin II receptor antagonist is lisinopril or ramaprilat.
52. Use according to any of embodiments 1-46, wherein said ACE inhibitor is ramiprilat or a pharmaceutically acceptable salt thereof.
53. Use according to any of embodiments 1-52, wherein said medicament comprises more than one ACE inhibitor or angiotensin II receptor antagonist, or pharmaceutically acceptable salt thereof.
54. Use according to any of embodiments 1-53, wherein said medicament contains 0.01-100 mg/kg ACE inhibitor and/or angiotensin II receptor antagonist.
55. Use according to embodiment 54, wherein said medicament contains 0.1-10 mg/kg ACE inhibitor and/or angiotensin II receptor antagonist.
56. Use according to any of embodiments 1-55, wherein said medicament further comprises a pharmaceutically-acceptable topical carrier.
57. Use according to any of embodiments 1-56, wherein said medicament further comprises one or more of a hormone, plant and/or herbal extracts, moisturizers or humectants, emollients, fragrances, sunscreen actives, anti-wrinkle and/or anti-ageing actives, whitening and/or bleaching actives, sunless tanning actives, preservative and/or antimicrobial actives, anti-acne actives, anti-psoriasis actives, anti-eczema actives, anti-inflammatory actives, vitamin actives, proteins, peptides, amino acids, amino aicd derivatives, insect repellants, fungicides, anti-viral agents, anti-cancer agents, anti-hemorrhoid compounds, anti-dandruff compounds, hair-growth stimulating compounds, hair-loss stimulating compounds, nucleic acids, chelating agents, pigments, lipids and/or inorganic salts.
58. Use according to any of embodiments 1-57, wherein said medicament further comprises one or more of a hydroxy acid, lactic acid, 2-hydroxy butanoic acid, malic acid, citric acid tartaric acid, decorin-synthesis enhancers, retinoids which include retinol and its esters, retinal, retinoic acid and its derivatives, retinoids, an alpha-hydroxy acid, a beta-hydroxy acid, an alpha-keto acids, a beta-keto acid, a peroxide, a vitamin, an anti-free-radical active agents, selenium, zinc, a beta-carotene, tensioning polymers of natural or synthetic origin, collagen-synthesis enhancers, a matrix metalloproteinase inhibitor, an antioxidant, a collagen modulator, an alpha-hydroxyethanoic acid, a hydroxycaprylic acid; an alpha-hydroxycarboxylic acid, beta-hydroxycarboxylic acid, a ceramide, a polyhydroxy acid, gamma-linolenic acid, a fruit acid, sugar cane extract, a skin peel agent, or a cosmeceutically-acceptable salt thereof.
59. Use according to any of embodiments 1-58, wherein said medicament is formulated in any suitable manner for application to an individual's skin.
60. Use according to any of embodiments 1-59, wherein said medicament is formulated as a lotion, cream, face mask, powder, skin patch, ointment, paste, water-based liquid, oil-based liquid or sprayable liquid.
61. Use according to any of embodiments 1-60, wherein said medicament is formulated as a cream or lotion.
62. The use according to any of embodiments 1-61, wherein the medicament is administered at least once daily
63. The use according to any of embodiments 1-62, wherein the medicament is administered at least two times daily.
64. The use according to any of embodiments 1-63, wherein the medicament is administered in a concentration equivalent of from 0.01 to 100 mg per kg.
65. The use according to any of embodiments 1-64, wherein the medicament is administered in a concentration equivalent of from 0.1 to 10 mg per kg.
66. A cosmetic method for improving and/or maintaining the skin tone of an individual suffering from, or at risk of suffering from, a dermatological disorder, said method comprising contacting the skin of said individual with an ACE inhibitor and/or angiotensin II receptor antagonist, or pharmaceutically acceptable salt thereof.
67. The cosmetic method according to embodiment 66, whereby the dermatological disorder is any of the disorders described in claims 2-46
68. The cosmetic method according to any of embodiments 66-67, wherein said contacting is conducted at least once daily.
69. The cosmetic method according to any of embodiments 66-68 wherein said method further comprises contacting the skin with one or more hormones, plant and/or herbal extracts, moisturizers or humectants, emollients, fragrances, sunscreen actives, anti-wrinkle and/or anti-ageing actives, whitening and/or bleaching actives, sunless tanning actives, preservative and/or antimicrobial actives, anti-acne actives, anti-psoriasis actives, anti-eczema actives, anti-inflammatory actives, vitamin actives, proteins, peptides, amino acids, amino aicd derivatives, insect repellants, fungicides, anti-viral agents, anti-cancer agents, anti-hemorrhoid compounds, anti-dandruff compounds, hair-growth stimulating compounds, hair-loss stimulating compounds, nucleic acids, chelating agents, pigments, lipids and/or inorganic salts.
70. The cosmetic method according to any of embodiments 66-69 which comprises repeatedly performing said contacting over an extended period of time.
71. The cosmetic method according to any of embodiments 66-70 which uses one or more ACE inhibitor or angiotensin II receptor antagonist according to embodiments 47-53.
72. The cosmetic method according to any of embodiments 66-71 using a formulation according to any of embodiments 54-61.
73. The cosmetic method according to any of embodiments 66-72 whereby the compounds are administered according to any of claims 62-65.

### Examples

### Example 1 - Preparation of a suitable cream base (O/W) for use with the ACE inhibitors and/or angiotensin II receptor antagonists of the present invention

| *Ingredients* | | |
|---|---|---|
| **Component** | % | **Function** |
| **I.** | | |
| Emulgade® SE | 4,0 | O/W cream base SE |
| Glyceryl Stearate (and) Ceteareth-20 (and) Ceteareth-12 (and) Cetearyl Alcohol (and) Cetyl Palmitate | | |
| Cutina® MD | 1,0 | Consistency giving factor |
| Glyceryl Stearate | | |
| Lanette® O | 1,0 | Consistency giving factor |
| Cetearyl Alcohol | | |
| Baysilon M 350 (Bayer) | 0,5 | Defoamer |
| Dimethicone | | |
| Cetiol® PGL | 7,0 | Emollient |
| Hexyldecanol (and) Hexyldecyl Laurate | | |
| Myritol® 312 | 3,0 | Emollient |
| Caprylic/Capric Triglyceride | | |
| Cetiol® OE | 4,0 | Emollient |
| Dicaprylyl Ether | | |
| Copherol® 1250 | 0,5 | active ingredient |
| Tocopheryl Acetate | | |

| **II.** | | |
|---|---|---|
| D-Panthenol (BASF) | 1,0 | active ingredient |
| Glycerin 86% | 5,0 | Moisturiser |
| Aqua | 71,5 | |

| **III.** | | |
|---|---|---|
| Carbopol 980 (Goodrich) | 0,2 | Stabiliser |
| Carbomer | | |
| Cetiol® PGL | 1,0 | Emollient |
| Hexyldecanol (and) Hexyldecyl Laurate | | |

| **IV.** | | |
|---|---|---|
| KOH, 20 % | 0,3 | Neutraliser |
| Perfume/preservative | n.B./q.s. | |
| Viscosity Brookfield, mPas | 100.000 | |
| RVF, 23 °C, Spindel/spindle TE, | | |
| 4 UpM/rpm, Helipath | | |

### Preparation

1. Melt the components listed under I at 80 - 85°C and stir until a homogeneous mixture results.
2. Heat the components listed under II to 80 - 85°C and add to phase I with stirring/homogenizing. Add phase III (Carbopol mixed with oil) into the hot emulsion and homogenise immediately by means of a suitable dispersion unit (Ultra Turrax). Allow the emulsion to cool with stirring in such a way that it remains in continual motion. Avoid the incorporation of air. Add the single components listed under IV at 40 °C. Allow to cool to 30° C.

### (All products in the text marked with an ® are trademarks of the Cognis group.)

### Example 2 - preparation of a suitable cream for topical application of an ACE inhibitor

The cream base described in Example 1 may be formulated with any ACE inhibitor. For example, a lisinopril cream may be formulated by adding 10 mg/kg Lisinopril to the cream base. Another example may be by adding 10 mg/kg Ramiprilat to the cream base.

### Example 3 - Trial demonstrating that the amount of fibrosis is reduced by local topical application of a skin lotion (crème) with the activated ACE-inhibitor Ramiprilat

### Trial Inclusion:

Females between 40 and 60 years.
More than 18 years
Can understand and sign informed consent.
Not pregnant or lactating
No known allergy to ACE-inhibitors.

### Method

A formulated skin lotion with or without a biologically amount of the active ACE-inhibitor Ramiprilat is applied to the dermis every morning on diseased areas and on non-diseased areas.

By mean of a 3-dimensional camera the amount of fibrosis on the diseased and non-diseased areas are digitalised.

The counting plan for digitalizing the fibrosis is as follow:

| Before | 2.wk | 4 wk | 8 wk | 12 wk | 16 wk | 20 wk | 24 wk |
|---|---|---|---|---|---|---|---|
| 0 | ½ | 1 | 2 | 3 | 4 | 5 | 6 |

The total number of counts are thus 8 per person in diseased areas and the same number in non-diseased areas.

40 evaluable patients is expected with a total number of counts of 640

It is also envisaged that biologically active amounts of other ACE inhibitors may be substituted in the same protocol.

## Claims

1. A medicament comprising an ACE inhibitor or pharmaceutically acceptable salt thereof and/or an angiotensin II receptor antagonist or pharmaceutically acceptable salt thereof for use in the treatment of a dermatological disorder, wherein said medicament is formulated for topical application and wherein said dermatological disorder is selected from the group consisting of:
a. psoriasis;
b. solar keratosis;
c. Scar formation;
d. Darier's disease;
e. pityriasis rubra pilaris;
f. dermatovenereal disease;
g. benign epidermal tumors;
h. malignant epidermal tumors;
i. adnexal tumors;
j. tumours of the sweat glands;
k. sebaceous tumors;
l. mesenchymal tumors;
m. melanocytic tumors;
n. Merkel cell carcinomas;
o. cysts of the skin and/or subcutis;
p. ichthyosis;
q. porokeratosis;
r. actinic cheilitis;
s. Bowen's intraepidermal carcinoma;
t. Bowen's disease;
u. Queyrath's disease;
v. Cornu cutaneum; and
w. basalioma

2. The medicament according to claim 1, wherein the dermatological disorder is psoriasis.

3. The medicament according to claim 1, wherein the dermatological disorder is solar keratosis.

4. The medicament according to claim 2, wherein said Psoriasis is selected from the group consisting of: psoriatic erytroderma, Palmoplantar psoriasis, palmoplantar pustulosis, generalized pustular psoriasis of Zumbusch and/or Lingua geographica.

5. The medicament according to any of the preceding claims, wherein said ACE inhibitor and/or angiotensin II receptor antagonist is selected from Alacepril, Delapril Benazepril, Cilazapril,Captopril, Enlapril, Fosinopril, Lisinopril, Moexipril, Perindopril, Ramipril, Pentopril, Zofenopril, Quinapril, Trandolapril, Imidapril, Isradipin, perindopril, spirapril, temocapril, Enalapril, losartan (Cozaar), valsartan (Diovan), irbesartan (Avapro), candesartan (Atacand), telmisartan (Micardis), eprosartan, tasosartan, zolarsartan, Zofenapril, Isradipin and Candesartancilexetil, or a pharmaceutically acceptable salt thereof.

6. The medicament according to any of claims 1 to 4, wherein said ACE inhibitor or angiotensin II receptor antagonist is selected from the group consisting of: captopril, quinaprilat, trandolaprilat, moexiprilat, fosinoprilat, fosinopril, benazeprilat, enalaprilat, ramaprilat and pharmaceutically acceptable salts thereof.

7. The medicament according to any of claims 1 to 4, wherein said ACE inhibitor or angiotensin II receptor antagonist is captopril or a pharmaceutically acceptable salt thereof.

8. The medicament according any of claims 1 to 4, wherein said ACE inhibitor or angiotensin II receptor antagonist is enalaprilat or a pharmaceutically acceptable salt thereof.

9. The medicament according to any of claims 1 to 8, wherein said medicament comprises more than one ACE inhibitor or angiotensin II receptor antagonist, or pharmaceutically acceptable salt thereof.

10. The medicament according to any of the preceding claims, wherein said medicament is formulated for administration of 0.01-100 mg ACE inhibitor and/or angiotensin II receptor antagonist per kg body weight.

11. The medicament according to any of the preceding claims, wherein said medicament is formulated in any suitable manner for application to an individual's skin.

12. The medicament according to any of the preceding claims, wherein said medicament is formulated as a lotion, cream, face mask, powder, skin patch, ointment, paste, water-based liquid, oil-based liquid or sprayable liquid.

13. The medicament according to any of the preceding claims, wherein said medicament is formulated as a cream, lotion or ointment.

14. The medicament according to any of the preceding claims, wherein the medicament is formulated for administration at least once daily

15. The medicament according to any of the preceding claims, wherein said medicament further comprises one or more selected from the group consisting of a hormone, plant extracts, herbal extracts, moisturizers, humectants, emollients, fragrances, sunscreen actives, anti-wrinkle actives, anti-ageing actives, whitening actives, bleaching actives, sunless tanning actives, preservative actives, antimicrobial actives, anti-acne actives, anti-psoriasis actives, anti-eczema actives, anti-inflammatory actives, vitamin actives, proteins, peptides, amino acids, amino aicd derivatives, insect repellants, fungicides, anti-viral agents, anti-cancer agents, anti-hemorrhoid compounds, anti-dandruff compounds, hair-growth stimulating compounds, hair-loss stimulating compounds, nucleic acids, chelating agents, pigments, lipids and inorganic salts.
